(19) Europäisches Patentamt
European Patent Office
Office européen des brevets

(11) EP 1 874 737 B1

(12) **EUROPÄISCHE PATENTSCHRIFT**

(45) Veröffentlichungstag und Bekanntmachung des Hinweises auf die Patenterteilung:
**04.08.2010 Patentblatt 2010/31**

(21) Anmeldenummer: **06723959.0**

(22) Anmeldetag: **01.04.2006**

(51) Int Cl.:
***C07D 235/28*** *(2006.01)* ***A61K 31/4184*** *(2006.01)*
***A61P 3/10*** *(2006.01)*

(86) Internationale Anmeldenummer:
**PCT/EP2006/003000**

(87) Internationale Veröffentlichungsnummer:
**WO 2006/111261 (26.10.2006 Gazette 2006/43)**

# (54) SUBSTITUIERTE 2-AMINOALKYLTHIO-BENZIMIDAZOLE UND IHRE VERWENDUNG ZUR BLUTZUCKERSENKUNG

SUBSTITUTED 2-AMINOALKYLTHIO-BENZIMIDAZOLES AND USE THEREOF FOR REDUCING BLOOD SUGAR LEVELS

BENZIMIDAZOLES 2-AMINOALKYLTHIO SUBSTITUES ET LEUR UTILISATION POUR DIMINUER LE TAUX DE SUCRE DANS LE SANG

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.04.2005 DE 102005017605**

(43) Veröffentlichungstag der Anmeldung:
**09.01.2008 Patentblatt 2008/02**

(73) Patentinhaber: **sanofi-aventis**
**75013 Paris (FR)**

(72) Erfinder:
- **DEFOSSA, Elisabeth**
  **65926 Frankfurt am Main (DE)**
- **SCHOENAFINGER, Karl**
  **65926 Frankfurt am Main (DE)**
- **JAEHNE, Gerhard**
  **65926 Frankfurt am Main (DE)**
- **BUNING, Christian**
  **65926 Frankfurt am Main (DE)**
- **TSCHANK, Georg**
  **65926 Frankfurt am Main (DE)**
- **WERNER, Ulrich**
  **65926 Frankfurt am Main (DE)**

(74) Vertreter: **Fischer, Hans-Jürgen et al**
**Sanofi-Aventis Deutschland GmbH**
**Patent- und Lizenzabteilung**
**Industriepark Höchst**
**Gebaüde K 801**
**65926 Frankfurt am Main (DE)**

(56) Entgegenhaltungen:
**WO-A-2005/021536**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 874 737 B1

Printed by Jouve, 75001 PARIS (FR)

## Beschreibung

Die Erfindung betrifft substituierte 2-Aminoalkylthio-benzimidazole sowie deren physiologisch verträgliche Salze.

Es sind bereits strukturähnliche Verbindungen im Stand der Technik beschrieben (siehe EP 1 122 257 und EP 0 392 317).

Der Erfindung lag die Aufgabe zugrunde, Verbindungen zur Verfügung zu stellen, die eine therapeutisch verwertbare Blutzucker senkende Wirkung entfalten.

Die Erfindung betrifft daher Verbindungen der Formel I,

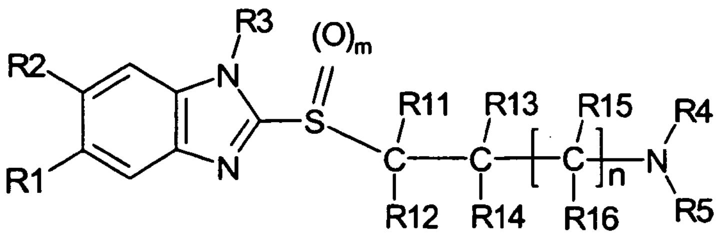

I

worin bedeuten

R1, R2 unabhängig voneinander H, CONR20R21, NR22COR23 oder NR24R25, wobei nicht beide Reste R1 oder R2 Wasserstoff entsprechen können;

R20 ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl oder ($C_1$-$C_6$)-Alkylen- ($C_6$-$C_{10}$)-Heterocyclyl, wobei die Alkyl-, Cycloalkyl-, Alkenyl-, Alkinyl-, Alkylen-, Aryl- und Heterocyclyl-Reste ein- oder mehrfach substituiert sein WO 2005/021536 offenbart Carbonitrilderivate, welche durch eine Benzimidazolgruppe substituiert sein können, und deren Verwendung als DPP-IV Inhibitoren, insbesondere zur Behandlung von Diabetes. können mit F, Cl, Br, I, CN, $NO_2$, SH, OH, ($C_1$-$C_6$)-Alkyl, O-($C_1$-$C_6$)-Alkyl, S- ($C_1$-$C_6$)-Alkyl, S(O)-($C_1$-$C_6$)-Alkyl oder $S(O)_2$-($C_1$-$C_6$)-Alkyl;

R21 H, ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl oder ($C_1$-$C_6$)-Alkylen- ($C_6$-$C_{10}$)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S- ($C_1$-$C_6$)-Alkyl, S(O)-($C_1$-$C_6$)-Alkyl oder S $(O)_2$-($C_1$-$C_6$)-Alkyl;

R22, R23 unabhängig voneinander H, ($C_1$-$C_{10}$)-Alkyl, ($C_3$-$C_{10}$)-Cycloalkyl, ($C_2$-$C_{10}$)- Alkenyl, ($C_2$-$C_{10}$)-Alkinyl, ($C_6$-$C_{10}$)-Aryl, Heterocyclyl, ($C_1$-$C_6$)-Alkylen-($C_6$- $C_{10}$)-Aryl, ($C_1$-$C_6$)-Alkylen-($C_6$-$C_{10}$)-Heterocyclyl oder S $(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Alkyl oder $S(O)_2$-($C_1$-$C_6$)-Alkyl;

R24 ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl, ($C_1$-$C_6$)-Alkylen-($C_6$- $C_{10}$)-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Alkyl oder $S(O)_2$-($C_1$-$C_6$)-Alkyl;

R25 H, ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl, ($C_1$-$C_6$)-Alkylen-($C_6$- $C_{10}$)-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Al-

kyl oder $S(O)_2$-($C_1$-$C_6$)-Alkyl;

R3 unabhängig voneinander $CF_3$, ($C_2$-$C_{10}$)-Akyl, ($C_3$-$C_{10}$)-Cycloakyl, ($C_2$-$C_{10}$)- Alkenyl, ($C_2$-$C_{10}$)-Alkinyl, ($C_6$-$C_{10}$)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, ($C_1$-$C_6$)- Alkyl, -$CF_3$, -$OCF_3$, -$SCF_3$, ($C_2$-$C_6$)-Alkenyl, ($C_2$-$C_6$)-Alkinyl, OR7, OP(O)$(OR7)_2$, NR7R8, NR7CONR7R8, COR7, OCOR7, OCOOR7, COOR7, CONR7R8, OCONR7R8, ($C_1$-$C_6$)-Alkylen-OR7, ($C_1$-$C_6$)-Alkylen-NR7R8, ($C_1$- $C_6$)-Alkylen-NR7$S(O)_2$R7, ($C_1$-$C_6$)-Alkylen-SR7, ($C_1$-$C_6$)-Alkylen-S(O)R7, ($C_1$- $C_6$)-Alkylen-$S(O)_2$R7, ($C_1$-$C_6$)-Alkylen-$S(O)_2$NR7R8, ($C_1$-$C_6$)-Alkylen-COR7, ($C_1$-$C_6$)-Alkylen-COOR7, ($C_1$-$C_6$)-Alkylen-CONR7R8, SR7, S(O)R7, $S(O)_2$R7, $S(O)_2$NR7R8, NR7$S(O)_2$R7, ($C_1$-$C_6$)-Akylen-($C_3$-$C_{10}$)-Cycloalkyl, ($C_1$-$C_6$)- Alkylen-($C_6$-$C_{10}$)-Aryl, ($C_1$-$C_6$)-Alkylen-Heterocyclyl, ($C_3$-$C_{10}$)-Cycloalkyl, ($C_6$- $C_{10}$)-Aryl oder Heterocyclyl;

wobei die Bedeutung unsubstituiertes oder substituiertes Piperidin-4-yl und unsubstituiertes oder substituiertes -$CH_2$-($C_6H_4$)-($C_6H_5$) ausgenommen sind.

R7, R8 unabhängig voneinander H, ($C_1$-$C_6$)-Alkyl, -$CF_3$, ($C_3$-$C_{10}$)-Cycloalkyl, ($C_6$-$C_{10}$)- Aryl, Heterocyclyl, ($C_1$-$C_6$)-Alkylen-CONR9R10, CONR9R10, ($C_1$-$C_6$)-Alkylen- COOR9, COOR9, COR9, ($C_1$-$C_6$)-Alkylen-COR9, ($C_1$-$C_6$)-Alkylen-OR9, ($C_1$- $C_6$)-Alkylen-NR9R10, ($C_1$-$C_6$)-Alkylen-SR9, ($C_1$-$C_6$)-Alkylen-S(O)R9, ($C_1$-$C_6$)- Alkylen-$S(O)_2$R9, S(O)R9, $S(O)_2$R9, ($C_1$-$C_4$)-Alkylen-($C_6$-$C_{10}$)-Aryl oder ($C_1$- $C_4$)-Alkylen-Heterocyclyl;

R9, R10 unabhängig voneinander H, ($C_1$-$C_6$)-Alkyl, ($C_1$-$C_6$)-Alkylen-($C_6$-$C_{10}$)-Aryl, -($C_6$-$C_{10}$)-Aryl, Heterocyclyl oder ($C_1$-$C_6$)-Akylen-Heterocyclyl;

R4, R5 unabhängig voneinander H, ($C_1$-$C_6$)-Alkyl oder ($C_3$-$C_8$)-Cykloalkyl, wobei ($C_1$- $C_6$)-Alkyl oder ($C_3$-$C_8$)-Cykloalkyl substitiuert sein können mit F, Cl, Br, I, CN, Aryl, Heterocyclyl, $NH_2$, NH ($C_1$-$C_6$)-Alkyl, N(($C_1$-$C_6$)-Alkyl$)_2$, OH, O($C_1$-$C_6$)- Alkyl, OAryl, OHeteroaryl, S($C_1$-$C_6$)-Alkyl, S(O)($C_1$-$C_6$)-Alkyl oder $S(O)_2$($C_1$-$C_6$)-Alkyl, wobei diese Alkylgruppen wiederum mit F, Cl, Br oder I substituiert sein können;

R11, R12, R13, R14, R15, R16 unabhängig voneinander H, ($C_1$-$C_6$)-Alkyl, Aryl, Heterocyclyl, ($C_3$-$C_8$)-Cykloalkyl, ($C_1$-$C_4$)-Alkylen-O-($C_1$-$C_6$)-Alkyl, ($C_1$-$C_4$)-Alkylen-S-($C_1$-$C_6$)-Akyl, ($C_1$-$C_4$)-Alkylen-NH-($C_1$-$C_6$)-Akyl, ($C_1$-$C_4$)-Alkylen- N(Alkyl$)_2$, ($C_1$-$C_4$)-Alkylen-($C_6$-$C_{10}$)-Aryl, ($C_1$-$C_4$)-Alkylen-Heterocyclyl, F, Cl, Br, I, CN, COOH, COO($C_1$-$C_6$)-Alkyl, $CONH_2$, CONH($C_1$-$C_6$)-Alkyl, CON(($C_1$- $C_6$)-Alkyl$)_2$, $CF_3$,

oder zwei der Reste R11, R12, R13, R14, R15, R16 bilden gemeinsam einen ($C_2$-$C_6$)- Alkylenrest, an dem ein ($C_6$-$C_{10}$)-Arylrest oder ein ($C_6$-$C_{10}$)-Heterocyclylrest ankondensiert sein kann und diese Arylreste oder Heterocyclykeste mit F, Cl, Br, I, $OCF_3$, $CF_3$, CN, ($C_1$-$C_6$)-Alkyl, Aryl, Heterocyclyl, ($C_3$-$C_8$)-Cykloalkyl, ($C_1$- $C_4$)-Alkylen-O-($C_1$-$C_6$)-Alkyl, ($C_1$-$C_4$)-Akylen-S-($C_1$-$C_6$)-Alkyl, ($C_1$-$C_4$)- Alkylen-NH-($C_1$-$C_6$)-Alkyl, ($C_1$-$C_4$)-Alkylen-N(($C_1$-$C_6$)-Alkyl$)_2$, ($C_1$-$C_4$)- Alkylen-($C_6$-$C_{10}$)-Aryl, ($C_1$-$C_4$)-Alkylen-Heterocyclyl, COOH, COO($C_1$-$C_6$)- Alkyl, $CONH_2$, CONH($C_1$-$C_6$)-Alkyl, CON(($C_1$-$C_6$)-Akyl$)_2$, OH, O-($C_1$-$C_6$)- Alkyl, O-($C_3$-$C_6$)-Cycloalkyl, S-($C_1$-$C_6$)-Alkyl, S-($C_3$-$C_6$)-Cycloalkyl, SO-($C_1$- $C_6$)-Alkyl, SO-($C_3$-$C_6$)-Cycloalkyl, $SO_2$-($C_1$-$C_6$)-Alkyl, $SO_2$-($C_3$-$C_6$)-Cycloalkyl, $SO_2$-$NH_2$, $SO_2$-NH-($C_1$-$C_6$)-Alkyl oder $SO_2$-NH-($C_3$-$C_7$)-Cycloalkyl substituiert sein können;

m 0, 1, oder 2;

n 0 oder 1;

sowie deren physiologisch verträglichen Salze.

Bevorzugt sind Verbindungen der Formel I, worin ein oder mehrere Reste die folgende Bedeutung haben:

R1, R2 unabhängig voneinander H, NR22COR23 oder NR24R25, wobei nicht beide Reste R1 oder R2 Wasserstoff entsprechen können;

R22, R23 unabhängig voneinander H, $(C_1-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, $(C_1-C_6)$-Alkylen-$(C_6- C_{10})$-Aryl, $(C_1-C_6)$-Alkylen-$(C_6-C_{10})$-Heterocyclyl oder S$(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder $S(O)_2$-$(C_1-C_6)$-Alkyl;

R24 $(C_1-C_{10})$-Alkyl, $(C_3-C_{10})$-Cycloalkyl, $(C_2-C_{10})$-Alkenyl, $(C_2-C_{10})$-Akinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder $S(O)_2$-$(C_1-C_6)$-Alkyl;

R25 H, $(C_1-C_{10})$-Alkyl, $(C_3-C_{10})$-Cycloalkyl, $(C_2-C_{10})$-Alkenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder $S(O)_2$-$(C_1-C_6)$-Alkyl;

R3 unabhängig voneinander $CF_3$, $(C_2-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, $(C_1-C_6)$- Alkyl, $-CF_3$, $-OCF_3$, $-SCF_3$, $(C_2-C_6)$-Alkenyl, $(C_2-C_6)$-Alkinyl, OR7, $OP(O)(OR7)_2$, NR7R8, NR7CONR7R8, COR7, OCOR7, OCOOR7, COOR7, CONR7R8, OCONR7R8, $(C_1-C_6)$-Alkylen-OR7, $(C_1-C_6)$-Alkylen-NR7R8, $(C_1- C_6)$-Alkylen-NR7$S(O)_2$R7, $(C_1-C_6)$-Alkylen-SR7, $(C_1-C_6)$-Alkylen-S(O)R7, $(C_1- C_6)$-Alkylen-$S(O)_2$R7, $(C_1-C_6)$-Alkylen-$S(O)_2$NR7R8, $(C_1-C_6)$-Alkylen-COR7, $(C_1-C_6)$-Alkylen-COOR7, $(C_1-C_6)$-Alkylen-CONR7R8, SR7, S(O)R7, $S(O)_2$R7, $S(O)_2$NR7R8, NR7$S(O)_2$R7, $(C_1-C_6)$-Alkylen-$(C_3-C_{10})$-Cycloalkyl, $(C_1-C_6)$- Alkylen-$(C_6-C_{10})$-Aryl, $(C_1-C_6)$-Alkylen-Heterocyclyl, $(C_3-C_{10})$-Cycloalkyl, $(C_6- C_{10})$-Aryl oder Heterocyclyl;

wobei die Bedeutung unsubstituiertes oder substituiertes Piperidin-4-yl und unsubstituiertes oder substituiertes $-CH_2-(C_6H_4)-(C_6H_5)$ ausgenommen sind.

R7, R8 unabhängig voneinander H, $(C_1-C_6)$-Alkyl, $-CF_3$, $(C_3-C_{10})$-Cycloalkyl, $(C_6-C_{10})$- Aryl, Heterocyclyl, $(C_1-C_6)$-Alkylen-CONR9R10, CONR9R10, $(C_1-C_6)$-Alkylen- COOR9, COOR9, COR9, $(C_1-C_6)$-Alkylen-COR9, $(C_1-C_6)$-Alkylen-OR9, $(C_1- C_6)$-Alkylen-NR9R10, $(C_1-C_6)$-Alkylen-SR9, $(C_1-C_6)$-Alkylen-S(O)R9, $(C_1-C_6)$- Alkylen-$S(O)_2$R9, S(O)R9, $S(O)_2$R9, $(C_1-C_4)$-Alkylen-$(C_6-C_{10})$-Aryl oder $(C_1- C_4)$-Alkylen-Heterocyclyl;

R9, R10 unabhängig voneinander H, $(C_1-C_6)$-Alkyl, $(C_1-C_6)$-Alkylen-$(C_6-C_{10})$-Aryl, $-(C_6- C_{10})$-Aryl, Heterocyclyl oder $(C_1-C_6)$-Alkylen-Heterocyclyl;

R4, R5 unabhängig voneinander H, $(C_1-C_6)$-Alkyl oder $(C_3-C_8)$-Cykloalkyl, wobei $(C_1- C_6)$-Alkyl oder $(C_3-C_8)$-Cykloalkyl substitiuert sein können mit F, Cl, Br, I, CN, Aryl, Heterocyclyl, $NH_2$, NH$(C_1-C_6)$-Alkyl, N($(C_1-C_6)$-Alkyl$)_2$, OH, O$(C_1-C_6)$- Alkyl, OAryl, OHeteroaryl, S$(C_1-C_6)$-Alkyl, S(O)$(C_1-C_6)$-Alkyl oder $S(O)_2(C_1-C_6)$-Alkyl, wobei diese Alkylgruppen wiederum mit F, Cl, Br oder I substituiert sein können;

| | |
|---|---|
| R11, R12, R13, R14, R15, R16 | unabhängig voneinander H, $(C_1-C_6)$-Alkyl, Aryl, Heterocyclyl, $(C_3-C_8)$-Cykloalkyl, $(C_1-C_4)$-Alkylen-O-$(C_1-C_6)$-Alkyl, $(C_1-C_4)$-Alkylen-S-$(C_1-C_6)$-Alkyl, $(C_1-C_4)$-Alkylen-NH-$(C_1-C_6)$-Alkyl, $(C_1-C_4)$-Alkylen- N(Alkyl)$_2$, $(C_1-C_4)$-Alkylen-$(C_6-C_{10})$-Aryl, $(C_1-C_4)$-Alkylen-Heterocyclyl, F, Cl, Br, I, CN, COOH, COO $(C_1-C_6)$-Alkyl, $CONH_2$, CONH$(C_1-C_6)$-Alkyl, CON($(C_1- C_6)$-Alkyl)$_2$, $CF_3$, |
| oder zwei der Reste R11, R12, R13, R14, R15, R16 | bilden gemeinsam einen $(C_2-C_6)$- Alkylenrest, an dem ein $(C_6-C_{10})$-Arylrest oder ein $(C_6-C_{10})$-Heterocyclylrest ankondensiert sein kann und diese Arylreste oder Heterocyclylreste mit F, Cl, Br, I, $OCF_3$, $CF_3$, CN, $(C_1-C_6)$-Alkyl, Aryl, Heterocyclyl, $(C_3-C_8)$-Cykloalkyl, $(C_1- C_4)$-Alkylen-O-$(C_1-C_6)$-Alkyl, $(C_1-C_4)$-Alkylen-S-$(C_1-C_6)$-Alkyl, $(C_1-C_4)$- Alkylen-NH-$(C_1-C_6)$-Alkyl, $(C_1-C_4)$-Alkylen-N($(C_1-C_6)$-Alkyl)$_2$, $(C_1-C_4)$- Alkylen-$(C_6-C_{10})$-Aryl, $(C_1-C_4)$-Alkylen-Heterocylyl, COOH, COO$(C_1-C_6)$- Alkyl, $CONH_2$, CONH$(C_1-C_6)$-Alkyl, CON($(C_1-C_6)$-Alkyl)$_2$, OH, O-$(C_1-C_6)$- Alkyl, O-$(C_3-C_6)$-Cycloalkyl, S-$(C_1-C_6)$-Alkyl, S-$(C_3-C_6)$-Cycloalkyl, SO-$(C_1-$ $C_6)$-Alkyl, SO-$(C_3-C_6)$-Cycloalkyl, $SO_2$-$(C_1-C_6)$-Alkyl, $SO_2$-$(C_3-C_6)$-Cycloalkyl, $SO_2$-$NH_2$, $SO_2$-NH-$(C_1-C_6)$-Alkyl oder $SO_2$-NH-$(C_3-C_7)$-Cycloalkyl substituiert sein können; |
| m | 0, 1, oder 2; |
| n | 0 oder 1; |

sowie deren physiologisch verträglichen Salze.

Besonders bevorzugt sind Verbindungen der Formel I, worin ein oder mehrere Reste die folgende Bedeutung haben:

| | |
|---|---|
| R1 | H; |
| R2 | NR22COR23 oder NR24R25; |
| R22, R23 | unabhängig voneinander H, $(C_1-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, $(C_1-C_6)$-Alkylen-$(C_6- C_{10})$-Aryl, $(C_1-C_6)$-Alkylen-$(C_6-C_{10})$-Heterocyclyl oder S(O)$_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder S(O)$_2$-$(C_1-C_6)$-Alkyl; |
| R24 | $(C_1-C_{10})$-Alkyl, $(C_3-C_{10})$-Cyloalkyl, $(C_2-C_{10})$-Akenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder S(O)$_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder S(O)$_2$-$(C_1-C_6)$-Alkyl; |
| R25 | H, $(C_1-C_{10})$-Alkyl, $(C_3-C_{10})$-Cycloalkyl, $(C_2-C_{10})$-Alkenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder S(O)$_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Akyl oder S(O)$_2$-$(C_1-C_6)$-Alkyl; |
| R3 | unabhängig voneinander $CF_3$, $(C_2-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, $(C_1-C_6)$- Alkyl, $-CF_3$, $-OCF_3$, $-SCF_3$, $(C_2-C_6)$-Alkenyl, $(C_2-C_6)$-Alkinyl, OR7, OP(O)(OR7)$_2$, NR7R8, NR7CONR7R8, COR7, OCOR7, OCOOR7, COOR7, CONR7R8, OCONR7R8, $(C_1-C_6)$-Alkylen-OR7, $(C_1-C_6)$-Alkylen-NR7R8, $(C_1- C_6)$-Al- |

kylen-NR7S(O)$_2$R7, ($C_1$-$C_6$)-Alkylen-SR7, ($C_1$-$C_6$)-Alkylen-S(O)R7, ($C_1$- $C_6$)-Alkylen-S(O)$_2$R7, ($C_1$-$C_6$)-Alkylen-S(O)$_2$NR7R8, ($C_1$-$C_6$)-Alkylen-COR7, ($C_1$-$C_6$)-Alkylen-COOR7, ($C_1$-$C_6$)-Alkylen-CONR7R8, SR7, S(O)R7, S(O)$_2$R7, S(O)$_2$NR7R8, NR7S(O)$_2$R7, ($C_1$-$C_6$)-Alkylen-($C_3$-$C_{10}$)-Cycloalkyl, ($C_1$-$C_6$)- Alkylen-($C_6$-$C_{10}$)-Aryl, ($C_1$-$C_6$)-Alkylen-Heterocyclyl, ($C_3$-$C_{10}$)-Cycloalkyl, ($C_6$- $C_{10}$)-Aryl oder Heterocyclyl;

wobei die Bedeutung unsubstituiertes oder substituiertes Piperidin-4-yl und unsubstituiertes oder substituiertes -$CH_2$-($C_6H_4$)-($C_6H_5$) ausgenommen sind.

| | |
|---|---|
| R7, R8 | unabhängig voneinander H, ($C_1$-$C_6$)-Alkyl, -$CF_3$, ($C_3$-$C_{10}$)-Cycloalkyl, ($C_6$-$C_{10}$)- Aryl, Heterocyclyl, ($C_1$-$C_6$)-Alkylen-CONR9-R10, CONR9R10, ($C_1$-$C_6$)-Alkylen- COOR9, COOR9, COR9, ($C_1$-$C_6$)-Alkylen-COR9, ($C_1$-$C_6$)-Alkylen-OR9, ($C_1$- $C_6$)-Alkylen-NR9R10, ($C_1$-$C_6$)-Alkylen-SR9, ($C_1$-$C_6$)-Alkylen-S(O)R9, ($C_1$-$C_6$)- Alkylen-S(O)$_2$R9, S(O)R9, S(O)$_2$R9, ($C_1$-$C_4$)-Alkylen-($C_6$-$C_{10}$)-Aryl oder ($C_1$- $C_4$)-Alkylen-Heterocyclyl; |
| R9, R10 | unabhängig voneinander H, ($C_1$-$C_6$)-Alkyl, ($C_1$-$C_6$)-Alkylen-($C_6$-$C_{10}$)-Aryl, -($C_6$- $C_{10}$)-Aryl, Heterocyclyl oder ($C_1$-$C_6$)-Alkylen-Heterocyclyl; |
| R4, R5 | unabhängig voneinander H, ($C_1$-$C_6$)-Alkyl oder ($C_3$-$C_8$)-Cykloalkyl, wobei ($C_1$- $C_6$)-Alkyl oder ($C_3$-$C_8$)-Cykloalkyl substitiuert sein können mit F, Cl, Br, I, CN, Aryl, Heterocyclyl, $NH_2$, NH($C_1$-$C_6$)-Alkyl, N(($C_1$-$C_6$)-Alkyl)$_2$, OH, O($C_1$-$C_6$)- Alkyl, OAryl, OHeteroaryl, S($C_1$-$C_6$)-Alkyl, S(O)($C_1$-$C_6$)-Alkyl oder S(O)$_2$($C_1$-$C_6$)-Alkyl, wobei diese Alkylgruppen wiederum mit F, Cl, Br oder I substituiert sein können; |
| R11, R12, R13, R14, R15, R16 | unabhängig voneinander H, ($C_1$-$C_6$)-Alkyl, Aryl, Heterocyclyl, ($C_3$-$C_8$)-Cykloalkyl, ($C_1$-$C_4$)-Alkylen-O-($C_1$-$C_6$)-Alkyl, ($C_1$-$C_4$)-Alkylen-S-($C_1$-$C_6$)-Alkyl, ($C_1$-$C_4$)-Alkylen-NH-($C_1$-$C_6$)-Alkyl, ($C_1$-$C_4$)-Alkylen- N(Alkyl)$_2$, ($C_1$-$C_4$)-Alkylen-($C_6$-$C_{10}$)-Aryl, ($C_1$-$C_4$)-Alkylen-Heterocyclyl, F, Cl, Br, I, CN, COOH, COO($C_1$-$C_6$)-Alkyl, $CONH_2$, CONH($C_1$-$C_6$)-Alkyl, CON(($C_1$- $C_6$)-Alkyl)$_2$, $CF_3$, |
| oder zwei der Reste R11, R12, R13, R14, R15, R16 | bilden gemeinsam einen ($C_2$-$C_6$)- Alkylenrest, an dem ein ($C_6$-$C_{10}$)-Arylrest oder ein ($C_6$-$C_{10}$)-Heterocyclylrest ankondensiert sein kann und diese Arylreste oder Heterocyclylreste mit F, Cl, Br, I, $OCF_3$, $CF_3$, CN, ($C_1$-$C_6$)-Alkyl, Aryl, Heterocyclyl, ($C_3$-$C_8$)-Cykloalkyl, ($C_1$- $C_4$)-Alkylen-O-($C_1$-$C_6$)-Alkyl, ($C_1$-$C_4$)-Alkylen-S-($C_1$-$C_6$)-Alkyl, ($C_1$-$C_4$)- Alkylen-NH-($C_1$-$C_6$)-Alkyl, ($C_1$-$C_4$)-Akylen-N(($C_1$-$C_6$)-Alkyl)$_2$, ($C_1$-$C_4$)- Alkylen-($C_6$-$C_{10}$)-Aryl, ($C_1$-$C_4$)-Alkylen-Heterocyclyl, COOH, COO($C_1$-$C_6$)- Alkyl, $CONH_2$, CONH($C_1$-$C_6$)-Alkyl, CON(($C_1$-$C_6$)-Alkyl)$_2$, OH, O-($C_1$-$C_6$)- Alkyl, O-($C_3$-$C_6$)-Cycloalkyl, S-($C_1$-$C_6$)-Alkyl, S-($C_3$-$C_6$)-Cycloalkyl, SO-($C_1$- $C_6$)-Alkyl, SO-($C_3$-$C_6$)-Cycloalkyl, $SO_2$-($C_1$-$C_6$)-Alkyl, $SO_2$-($C_3$-$C_6$)-Cycloalkyl, $SO_2$-$NH_2$, $SO_2$-NH-($C_1$-$C_6$)-Alkyl oder $SO_2$-NH-($C_3$-$C_7$)-Cycloalkyl substituiert sein können; |
| m | 0; |
| n | 0 oder 1; |

sowie deren physiologisch verträglichen Salze.

Die Erfindung bezieht sich auf Verbindungen der Formel I, in Form ihrer Razemate, razemischen Mischungen und reinen Enantiomeren sowie auf ihre Diastereomeren und Mischungen davon.

Können Reste oder Substituenten mehrfach in den Verbindungen der Formel I auftreten, so können sie alle unabhängig voneinander die angegebenen Bedeutungen haben und gleich oder verschieden sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin, oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette mit einem oder mehreren Kohlenstoffen verstanden, wie z.B. Methyl, Ethyl, iso-Propyl, tert.-Butyl, Hexyl.

Die Alkylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2NH$($C_1$-$C_6$)-Alkyl, $SO_2N$[($C_1$-$C_6$)-Alkyl]$_2$ , S-($C_1$-$C_6$)-Alkyl, S-($CH_2$)$_n$-Aryl, S-($CH_2$)$_n$-Heterocyclus, SO-($C_1$-$C_6$)-Alkyl, SO-($CH_2$)$_n$-Aryl, SO-($CH_2$)$_n$-Heterocyclus, $SO_2$-($C_1$-$C_6$)-Alkyl, $SO_2$-($CH_2$)$_n$-Aryl, $SO_2$-($CH_2$)$_n$-Heterocyclus, $SO_2$-NH($CH_2$)$_n$-Aryl, $SO_2$-NH($CH_2$)$_n$-Heterocyclus, $SO_2$-N(($C_1$-$C_6$)-Alkyl)($CH_2$)$_n$-Aryl, $SO_2$-N(($C_1$-$C_6$)-Alkyl)($CH_2$)$_n$-Heterocyclus, $SO_2$-N(($CH_2$)$_n$-Aryl)$_2$, $SO_2$-N(($CH_2$)$_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$substituiertseinkann;C(=NH)($NH_2$),$NH_2$,NH-($C_1$-$C_6$)-Alkyl,N(($C_1$-$C_6$)-Alkyl)$_2$,NH($C_1$-$C_7$)-Acyl,NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-Aryl, $SO_2$-NH($CH_2$)$_n$-Heterocyclus, $SO_2$-N(($C_1$-$C_6$)-Alkyl)($CH_2$)$_n$-Aryl, $SO_2$-N(($C_1$-$C_6$)-Alkyl)($CH_2$)$_n$-Heterocyclus, $SO_2$-N(($CH_2$)$_n$-Aryl)$_2$, $SO_2$-N(($CH_2$)$_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$ substituiert sein kann; C(=NH)($NH_2$), $NH_2$, NH-($C_1$-$C_6$)-Alkyl, N(($C_1$-$C_6$)-Alkyl)$_2$, NH($C_1$-$C_7$)-Acyl, NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl,NH-CO-NH-Aryl,NH-CO-NH-Heterocyclus,N(($C_1$-$C_6$)-Alkyl)-CO-($C_1$-$C_6$)-Alkyl,N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Akyl), N(($C_1$-$C_6$)-Akyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$- $C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N(Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N (Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-($CH_2$)$_n$-Aryl und O-($CH_2$)$_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Akyl oder $CONH_2$.

Unter einem Alkenylrest wird eine geradkettige oder verzweigte Kohlenwasserstoffkette mit zwei oder mehreren Kohlenstoffen sowie einer oder mehreren Doppelbindungen verstanden, wie z.B. Vinyl, Allyl, Pentenyl, 2-Methyl-but-2-en-4-yl.

Die Alkenylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Akyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2NH$($C_1$-$C_6$)-Alkyl, $SO_2N$[($C_1$-$C_6$)-Akyl]$_2$, S-($C_1$-$C_6$)-Alkyl, S-($CH_2$)$_n$-Aryl, S-($CH_2$)$_n$-Heterocyclus, SO-($C_1$-$C_6$)-Alkyl, SO-($CH_2$)$_n$-Aryl, SO-($CH_2$)$_n$-Heterocyclus, $SO_2$-($C_1$-$C_6$)-Alkyl, $SO_2$-($CH_2$)$_n$-Aryl, $SO_2$-($CH_2$)$_n$-Heterocyclus, $SO_2$-NH($CH_2$)$_n$-Aryl, $SO_2$-NH($CH_2$)$_n$-Heterocyclus, $SO_2$-N(($C_1$-$C_6$)-Alkyl)($CH_2$)$_n$-Aryl, $SO_2$-N(($C_1$-$C_6$)-Akyl)

$(CH_2)_n$-Heterocydus, $SO_2$-N($(CH_2)_n$-Aryl)$_2$, $SO_2$-N($(CH_2)_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$substituiertseinkann;C(=NH)($NH_2$),$NH_2$,NH-($C_1$-$C_6$)-Alkyl,N(($C_1$-$C_6$)-Alkyl)$_2$,NH($C_1$-$C_7$)-Acyl,NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Akyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$- $C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N (Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N (Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N (Aryl)-CO-N-(Aryl)$_2$, N(Heterocyclus)-CO-N-(Aryl)$_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Unter einem Alkinylrest wird eine geradkettige oder verzweigte Kohlenwasserstoffkette mit zwei oder mehreren Kohlenstoffen sowie einer oder mehreren Dreifachbindungen verstanden, wie z.B. Ethinyl, Propinyl, Butinyl, Hexinyl.

Die Alkinylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$, S-($C_1$-$C_6$)-Alkyl, S-$(CH_2)_n$-Aryl, S-$(CH_2)_n$-Heterocyclus, SO-($C_1$-$C_6$)-Alkyl, SO-$(CH_2)_n$-Aryl, SO-$(CH_2)_n$-Heterocyclus, $SO_2$-($C_1$-$C_6$)-Alkyl, $SO_2$-$(CH_2)_n$-Aryl, $SO_2$-$(CH_2)_n$-Heterocyclus, $SO_2$-NH$(CH_2)_n$-Aryl, $SO_2$-NH$(CH_2)_n$-Heterocyclus, $SO_2$-N(($C_1$-$C_6$)-Alkyl)$(CH_2)_n$-Aryl, $SO_2$-N(($C_1$-$C_6$)-Alkyl) $(CH_2)_n$-Heterocyclus, $SO_2$-N($(CH_2)_n$-Aryl)$_2$, $SO_2$-N($(CH_2)_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$substituiertseinkann;C(=NH)($NH_2$),$NH_2$,NH-($C_1$-$C_6$)-Alkyl,N(($C_1$-$C_6$)-Alkyl)$_2$,NH($C_1$-$C_7$)-Acyl,NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)- CO-($C_1$-$C_6$)-Alkyl, N (Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N (Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N (Aryl)-CO-N-(Aryl)$_2$, N(Heterocyclus)-CO-N-(Aryl)$_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$ , S-($C_1$-$C_6$)-Alkyl, S-$(CH_2)_n$-Aryl, S-$(CH_2)_n$-Heterocyclus, SO-($C_1$-$C_6$)-Alkyl, SO-$(CH_2)_n$-Aryl, SO-$(CH_2)_n$-Heterocyclus, $SO_2$-($C_1$-$C_6$)-Alkyl, $SO_2$-$(CH_2)_n$-Aryl, $SO_2$-$(CH_2)_n$-Heterocyclus, $SO_2$-NH$(CH_2)_n$-Aryl, $SO_2$-NH$(CH_2)_n$-Heterocyclus, $SO_2$-N(($C_1$-$C_6$)-Alkyl)$(CH_2)_n$-Aryl, $SO_2$-N(($C_1$-$C_6$)-Alkyl) $(CH_2)_n$-Heterocyclus, $SO_2$-N($(CH_2)_n$-Aryl)$_2$, $SO_2$-N($(CH_2)_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$substituiertseinkann;C(=NH)($NH_2$),$NH_2$,NH-($C_1$-$C_6$)-Alkyl,N(($C_1$-$C_6$)-Alkyl)$_2$,NH($C_1$-$C_7$)-Acyl,NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Alkyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Al-

kyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)- CO-($C_1$-$C_6$)-Alkyl, N (Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N (Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N (Aryl)-CO-N-(Aryl)$_2$, N(Heterocyclus)-CO-N-(Aryl)$_2$, Aryl, O-($CH_2$)$_n$-Aryl und O-($CH_2$)$_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Akyl]$_2$, Cycloalkyl, ($C_2$-$C_6$)-Alkenyl, ($C_2$-$C_6$)-Afidnyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$ , S-($C_1$-$C_6$)-Akyl, S-($CH_2$)$_n$-Aryl, S-($CH_2$)$_n$-Heterocyclus, SO-($C_1$-$C_6$)-Alkyl, SO-($CH_2$)$_n$-Aryl, SO-($CH_2$)$_n$-Heterocyclus, $SO_2$-($C_1$-$C_6$)-Alkyl, $SO_2$-($CH_2$)$_n$-Aryl, $SO_2$-($CH_2$)$_n$-Heterocyclus, $SO_2$-NH($CH_2$)$_n$-Aryl, $SO_2$-NH($CH_2$)$_n$-Heterocyclus, $SO_2$-N(($C_1$-$C_6$)-Alkyl)($CH_2$)$_n$-Aryl, $SO_2$-N((C1-$C_6$)-Alkyl) ($CH_2$)$_n$-Heterocyclus, $SO_2$-N(($CH_2$)$_n$-Aryl)$_2$, $SO_2$-N(($CH_2$)$_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$substituiertseinkann;C(=NH)($NH_2$),$NH_2$,NH-($C_1$-$C_6$)-Alkyl,N(($C_1$-$C_6$)-Alkyl)$_2$,NH($C_1$-$C_7$)-Acyl,NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Akyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$-$C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)- CO-($C_1$-$C_6$)-Alkyl, (Heterocyclus)-CO-($C_1$-$C_6$)-Alkyl, N(Aryl)-COO-($C_1$-$C_6$)-Alkyl, N(Heterocyclus)-COO-($C_1$-$C_6$)-Alkyl, N(Aryl)-CO-Aryl, N (Heterocyclus)-CO-Aryl, N(Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N(Aryl)-CO-NH-Aryl, N(Heterocyclus)-CO-NH-Aryl, N(Aryl)-CO-N(($C_1$-$C_6$)-Akyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N (Aryl)-CO-N-(Aryl)$_2$, N(Heterocyclus)-CO-N-(Aryl)$_2$, Aryl, O-($CH_2$)$_n$-Aryl und O-($CH_2$)$_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$ $SO_2$-$CH_3$, COOH, COO-($C_1$-$C_6$)-Alkyl oder $CONH_2$.

Unter Heterocyclyl, Heterocyclus bzw. heterocyclischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit Benzolkernen kondensiert ist. Der Heterocyclus bzw. der heterocyclische Rest kann aromatisch, gesättigt aliphatisch oder teilweise ungesättigt aliphatisch sein.

Geeignete Heterocyclyl-Reste, bzw. "Heterocyclische Reste" sind Acridinyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Umfasst sind weiterhin die entsprechenden N-Oxide dieser Verbindungen, also z.B. 1-Oxy 2-, 3- oder 4-Pyridyl.

Umfasst sind weiterhin ein oder mehrfach benzoannelierte Derivate dieser Heterocyclen.

Die Heterocyclischen Ringe bzw. Heterocyclische Reste können ein oder mehrfach mit geeigneten Gruppen

substituiert sein, wie z.B: F, Cl, Br, I, $CF_3$, $NO_2$, $N_3$, CN, COOH, COO($C_1$-$C_6$)Alkyl, $CONH_2$, CONH($C_1$-$C_6$)Alkyl, CON[($C_1$-$C_6$)Alkyl]$_2$, Cycloalkyl, ($C_2$-$C_6$)-Alkenyl, ($C_2$-$C_6$)-Alkinyl, O-($C_1$-$C_6$)-Alkyl, O-CO-($C_1$-$C_6$)-Alkyl, O-CO-($C_1$-$C_6$)-Aryl, O-CO-($C_1$-$C_6$)-Heterocyclus; $PO_3H_2$, $SO_3H$, $SO_2$-$NH_2$, $SO_2$NH($C_1$-$C_6$)-Alkyl, $SO_2$N[($C_1$-$C_6$)-Alkyl]$_2$ S-($C_1$-$C_6$)-Alkyl, S-$(CH_2)_n$-Aryl, S-$(CH_2)_n$-Heterocyclus, SO-($C_1$-$C_6$)-Alkyl, SO-$(CH_2)_n$-Aryl, SO-$(CH_2)_n$-Heterocyclus, $SO_2$-($C_1$-$C_6$)-Alkyl, $SO_2$-$(CH_2)_n$-Axyl, $SO_2$-$(CH_2)_n$-Heterocyclus, $SO_2$-NH($CH_2$)n-Aryl, $SO_2$-NH$(CH_2)_n$-Heterocyclus, $SO_2$-N(($C_1$-$C_6$)-Alkyl)$(CH_2)_n$Aryl, $SO_2$-N(($C_1$-$C_6$)-Alkyl)$(CH_2)_n$-Heterocyclus, $SO_2$-N($(CH_2)_n$-Aryl)$_2$, $SO_2$-N($(CH_2)_n$-Heterocyclus)$_2$ wobei n = 0 - 6 sein kann und der Arylrest oder Heterocyclische Rest bis zu zweifach mit F, Cl, Br, OH, $CF_3$, $NO_2$, CN, $OCF_3$, O-($C_1$-$C_6$)-Alkyl, ($C_1$-$C_6$)-Alkyl oder $NH_2$ substituiert sein kann; C(=NH)($NH_2$), $NH_2$, NH-($C_1$-$C_6$)-Alkyl, N(($C_1$-$C_6$)-Alkyl)$_2$, NH($C_1$-$C_7$)-Acyl, NH-COO-($C_1$-$C_6$)-Alkyl, NH-CO-Aryl, NH-CO-Heterocyclus, NH-COO-Aryl, NH-COO-Heterocyclus, NH-CO-NH-($C_1$-$C_6$)-Alkyl, NH-CO-NH-Aryl, NH-CO-NH-Heterocyclus, N(($C_1$-$C_6$)-Akyl)-CO-($C_1$-$C_6$)-Alkyl, N(($C_1$-$C_6$)-Alkyl)-COO-($C_1$-$C_6$)-Alkyl, N(($C_1$-$C_6$)-Alkyl)-CO-Aryl, N(($C_1$-$C_6$)-Alkyl)-CO-Heterocyclus, N(($C_1$-$C_6$)-Alkyl)-COO-Aryl, N(($C_1$-$C_6$)-Alkyl)-COO-Heterocyclus, N(($C_1$-$C_6$)-Alkyl)-CO-NH-($C_1$-$C_6$)-Alkyl), N(($C_1$- $C_6$)-Alkyl)-CO-NH-Aryl, N(($C_1$-$C_6$)-Alkyl)-CO-NH-Heterocyclus, N(($C_1$-$C_6$)-Alkyl)-CO-N(($C_1$-$C_6$)-Alkyl)$_2$, N(($C_1$-$C_6$)-Alkyl)-CO-N(($C_1$-$C_6$)-Alkyl)-Aryl, N(($C_1$-$C_6$)-Alkyl)-CO-N(($C_1$-$C_6$)-Alkyl)-Heterocyclus, N(($C_1$-$C_6$)-Alkyl)-CO-N-(Aryl)$_2$, N(($C_1$-$C_6$)-Alkyl)-CO-N-(Heterocyclus)$_2$, N(Aryl)-CO-($C_1$-$C_6$)-Alkyl, N(Heterocyclus)-CO-($C_1$-$C_6$)-Alkyl, N (Aryl)- COO-($C_1$-$C_6$)-Alkyl, N(Heterocyclus)-COO-($C_1$-$C_6$)-Alkyl, N(Aryl)-CO-Aryl, N(Heterocyclus)-CO-Aryl, N (Aryl)-COO-Aryl, N(Heterocyclus)-COO-Aryl, N(Aryl)-CO-NH-($C_1$-$C_6$)-Alkyl), N(Heterocyclus)-CO-NH-($C_1$-$C_6$)-Alkyl), N (Aryl)-CO-NH-Aryl, N(Heterocyclus)-CO-NH-Aryl, N(Aryl)-CO-N(($C_1$-$C_6$)-Alkyl)$_2$, N(Heterocyclus)-CO-N(($C_1$-$C_6$)-Alkyl)$_2$, N(Aryl)-CO-N(($C_1$-$C_6$)-Alkyl)-Aryl, N(Heterocyclus)-CO-N(($C_1$-$C_6$)-Alkyl)-Aryl, N(Aryl)-CO-N-(Aryl)$_2$, N(Heterocyclus)-CO-N-(Aryl)$_2$, Aryl, O-$(CH_2)_n$-Aryl und O-$(CH_2)_n$-Heterocyclus, wobei n = 0 - 6 sein kann, wobei der Arylrest oder Heterocyclische Rest ein bis 3-fach substituiert sein kann mit F, Cl, Br, I, OH, $CF_3$, $NO_2$, CN, $OCF_3$, O-($C_1$-$C_6$)-Alkyl, ($C_1$-$C_6$)-Alkyl, $NH_2$, NH($C_1$-$C_6$)-Alkyl, N(($C_1$-$C_6$)-Alkyl)$_2$, $SO_2$-$CH_3$, COOH, COO-($C_1$-$C_6$)-Alkyl oder $CONH_2$.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 ng pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt,

falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2005, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin oder Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, oder mit einer Verbindung, wie in PCT/EP

2004/00269, PCT/EP 2003/05815, PCT/EP 2003/05814, PCT/EP 2003/05816, EP 0114531 oder US 6,498,156 beschrieben, verabreicht.

Bei einer Ausführungsform der Erfindung werden die Verbindungen der Formel I in Kombination mit einem PPAR gamma Agonist, wie z.B. Rosiglitazon, Pioglitazon, JTT-501 oder Gl 262570, verabreicht.

Bei einer Ausführungsform der Erfindung werden die Verbindungen der Formel I in Kombination mit PPAR alpha Agonist, wie z.B. GW 9578 oder GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490 oder DE10142734.4 beschrieben verabreicht.

Bei einer Ausführungsform der Erfindung werden die Verbindungen der Formel I in Kombination mit einem Fibrat, wie z.B. Fenofibrat, Clofibrat oder Bezafibrat, verabreicht.

Bei einer Ausführungsform der Erfindung werden die Verbindungen der Formel I in Kombination mit einem MTP-Inhibitor, wie z.B. Implitapide , BMS-201038 oder R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897) verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin oder Colesevelam, verabreicht.

Bei einer Ausführungsform der Erfindung werden die Verbindungen der Formel I in Kombination mit einem LDL-Rezeptorinducer (siehe US 6,342,512) verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) Antagonisten, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid, verabreicht. Bei einer Ausführungsform werden die Verbindungen der Formel I in Kombination mit einem Biguanid, wie z.B. Metformin, verabreicht.

Bei einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem $\alpha$-Glukosidase-Inhibitor, wie z.B. Miglitol oder Acarbose, verabreicht.

Bei einer Ausführungsform werden die Verbindungen der Formel I in Kombination mit einem Adenosin A1 Agonisten, wie z. B. solchen, die in EP 0912520 oder PCT/EP06749 beschrieben sind, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc., verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure- {4-[(4-amino-quinazolin-2-ylamino)-methyl]-cyclohexylmethyl}-amid Hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid, (WO 01/91752)), Orexin-Antagonisten (z.B. 1-(2-Methyl-benzoxazol-6-yl)-3-[1,5]naphthyridin-4-yl-harnstoff Hydrochlorid (SB-3 34867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)), TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451)), CB1 (Cannabinoid Rezeptor 1) Rezeptor Antagonisten (z.B. Rimonabant oder die in WO 02/28346 genannten Wirkstoffe), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl} - essigsäure Trifluoressigsäuresalz (WO 99/15525)), Serotonin-Wiederaufnahme-Inhibitoren (z.B. Dexfenfluramine), gemischte Sertonin- und noradrenerge Verbindungen (siehe z.B. WO 00/71549), 5HT-Agonisten z.B. 1-(3-Ethyl-benzofuran-7-yl)-piperazin Oxalsäuresalz (WO 01/09111)), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)), TRH-Agonisten (siehe z.B. EP 0 462 884), entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881), DA-Agonisten (z.B. Bromocriptin oder Doprexin), Lipase/Amylase-Inhibitoren (siehe z.B. WO 00/40569), PPAR-Modulatoren (siehe z.B. WO 00/78312), RXR-Modulatoren oder TR-β-Agonisten verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer weiteren Ausführungsform ist der weitere Wirkstoff Rimonabant.

Bei einer Ausführungsform werden die Verbindungen der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

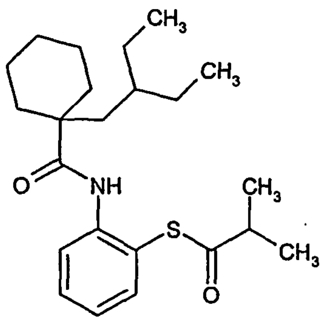

JTT-705

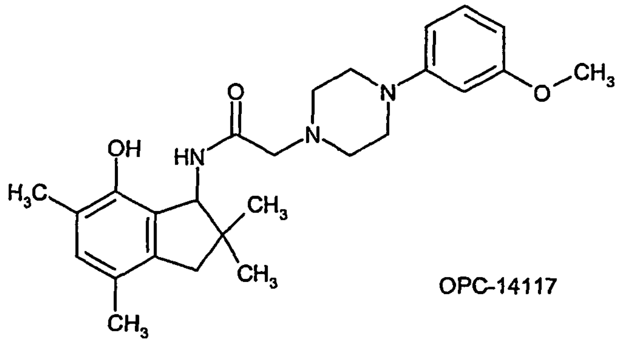

OPC-14117

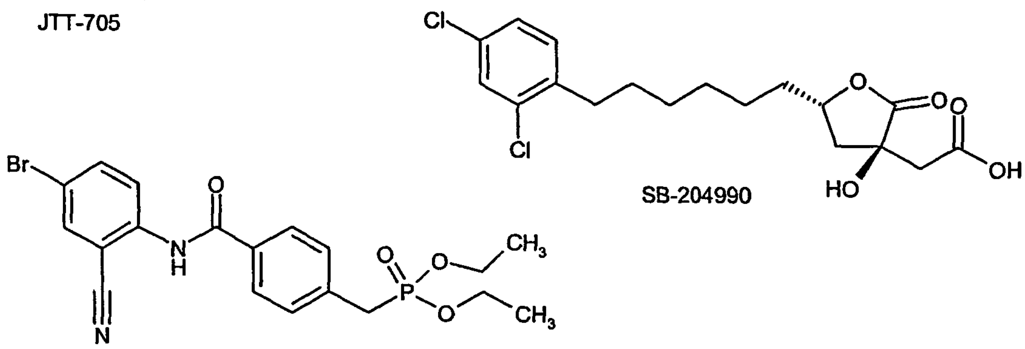

SB-204990

NO-1886

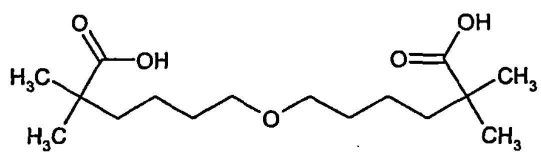

CI-1027

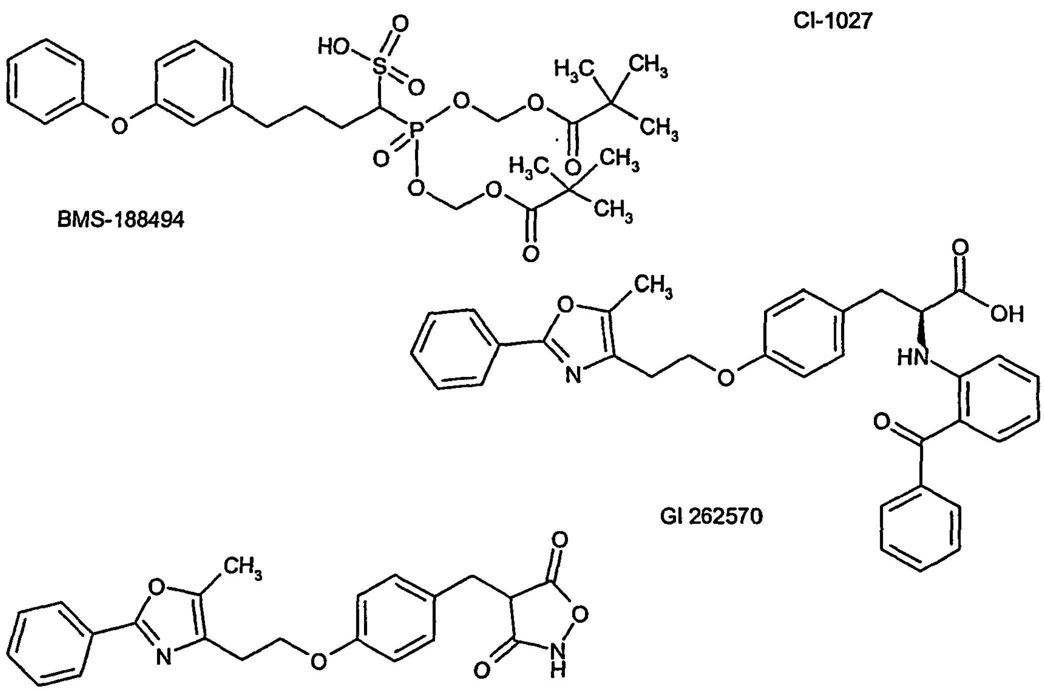

BMS-188494

GI 262570

JTT-501

Die Verbindungen der Formel I lassen sich dadurch herstellen, dass man geeignete Ausgangsstoffe der Formel II, worin R1, R2 und R3 die oben angegebene Bedeutung besitzen und X eine Austrittsgruppe, wie Chlor, Brom, Jod,

Sulfonyloxid, Sulfinyl oder Sulfoxyl, bedeutet, mit einer Verbindung der Formel V ggf. in Gegenwart von geeigneten Basen zu den Verbindungen der Formel IV umsetzt.

Alternativ setzt man Verbindungen der Formel III, worin R1, R2 und R3 die oben angegebene Bedeutung besitzen, mit Alkylierungsmittel der Formel VI zu den Verbindungen der Formel IV um, wobei X für eine geeignete Austrittsgruppe wie beispielsweise Chlor, Brom, Jod, Sulfonyloxi, Sulfinyl oder Sulfoxyl steht.

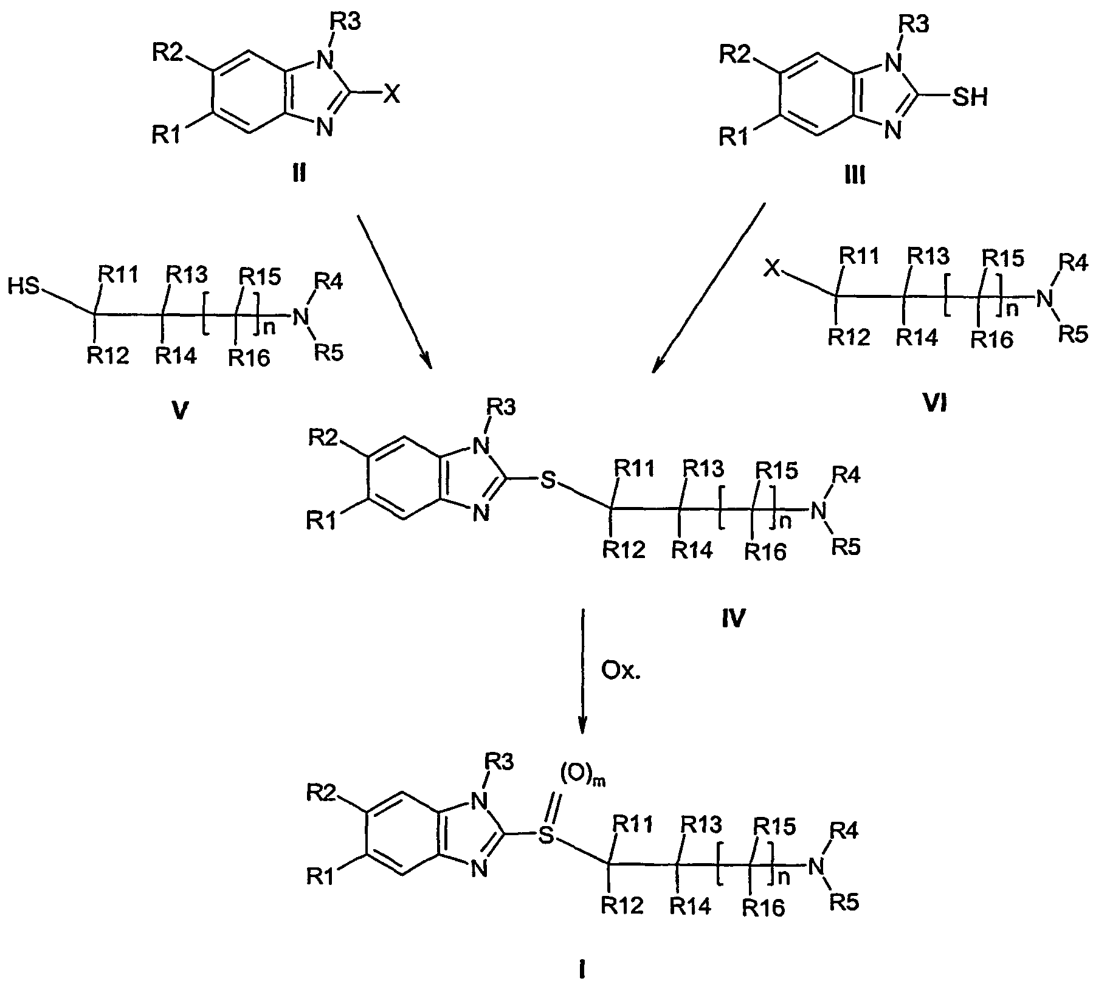


II

III

V

VI

IV

I

In den Fällen, wo R4 oder R5 Wasserstoff bedeutet, kann es zweckmäßig sein, den Rest - NR4R5 in an der Stickstofffunktion geschützter Form einzusetzen und die Schutzgruppe an geeigneter Stelle der Reaktion wieder abzuspalten. Solche geeigneten Schutzgruppen und die Verfahren der Einführung und Abspaltung sind bekannt (siehe: Theodora W. Greene and Peter G. M. Wuts, Protective Groups in Organic Synthesis, 3rd Edition, John Wiley & Sons, Inc., New York, 1999).

Die Thioetherfunktion in IV kann dann nach bekannten Methoden oxidiert werden zu den erfindungsgemäßen Stoffen der Formel I, worin n eine Zahl 1 oder 2 bedeutet.

Die Halogenverbindungen der Formel II können nach bekannten Verfahren wie beispielsweise durch Halogenierung der entsprechenden H-Verbindung (Formel II, X = H) erhalten werden. Geeignete Halogenierungsmittel können beispielhaft Halogene, wie Chlor und Brom, N-Bromsuccinimid, Phosphorpentoxid oder Phosphoroxichlorid sein.

Die Synthese von Verbindungen der Formel II ist in der Literatur beschrieben. Sie können beispielsweise durch Kondensation von substituierten Diaminobenzolderivaten mit Aldehyden in Gegenwart eines Oxidationsmitteln (z.B. Luftsauerstoff, Sauerstoff, Iod, Oxone, Chinonen, Peroxiden, usw.) oder alternativ mit Carbonsäuren, Nitrilen oder Amiden ohne oder in Gegenwart eines Katalysators hergestellt werden.

Die nachfolgend aufgeführten Beispiele dienen zur Erläuterung der Erfindung, ohne diese jedoch einzuschränken.

Tabelle 1:

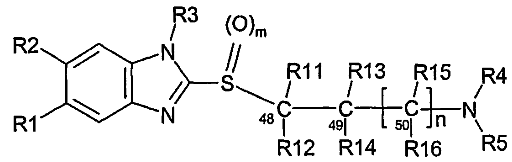

I

| Bsp. | R1 | R2 | R3 | m | R4 | R5 | R11 | R12 | R13 | R14 | n | R15 | R16 |
|---|---|---|---|---|---|---|---|---|---|---|---|---|---|
| 1 | H | Ph-CO-NH- | $-CH_2CH=C(CH_3)_2$ | 0 | H (TFA-Salz) | H | H | H | H | H | 1 | H | H |
| 2 | H | Ph-$CH_2$-NH- | $-CH_2CH=C(CH_3)_2$ | 0 | H (TFA-Salz) | H | H | H | H | H | 1 | H | H |
| 3 | H | Ph-CO-NH- | $-CH_2CH=C(CH_3)_2$ | 0 | H (TFA-Salz) | H | H | H | H | H | 0 | - | - |

Die Verbindungen der Formel I zeichnen sich durch günstige Wirkungen auf den Lipid- und Kohlenhydratstoffwechsel aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischen Erscheinungen geeignet. Die Verbindungen können allein oder in Kombination mit weiteren Blutzucker senkenden Wirkstoffen eingesetzt werden. Die Verbindungen wirken als DPP-IV (Dipeptidyl Peptidase IV) Inhibitoren und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus, zur Gewichtsreduktion bei Säugetieren, zur Behandlung von Immunstörungen und zur Behandlung von Drogenmissbrauch.

Weiterhin eignen sie sich zur Behandlung von Krebs, Arthritis, Osteoarthritis, Osteoporose, Schlafstörungen, Schlaf Apnoe, weiblicher und männlicher Sexualstörungen, Entzündungen, Akne, Pigmentierung der Haut, Störungen des Steroidstoffwechsels, Hautkrankheiten, Psoriasis, Mykosen, neurodegenerativer Krankheiten, Multiple Sklerose und Alzheimer-Krankheit.

Die Wirksamkeit der Verbindungen wurde wie folgt getestet:

Messung der DPP-IV Aktivität:

Material:

DPP-IV aus Schweineniere (Sigma, München)
H-Ala-Pro-AFC (Bachem, Weil am Rhein)

Testbedingungen:

DPP-IV (1 mU/ml, Endkonzentration)
H-Ala-Pro-AFC (15 μM Endkonzentration)
in Tris/HCl (40 mM, pH 7.4), Gesamtvolumen 0,2 ml

Die Reaktion wurde bei Raumtemperatur für unterschiedliche Zeiträume (typischerweise 10 Minuten) durchgeführt und am Ende der Reaktion durch Zugabe von 20 μl $ZnCl_2$ (1 M) gestoppt. Der Umsatz von H-Ala-Pro-AFC wurde fluorimetrisch durch Messung der Emission bei 535 nm nach Anregung bei 405 nm bestimmt. Im Falle der Zugabe von Inhibitoren wurde das zugegebene Puffervolumen so angepasst, dass ein Gesamtvolumen der Testmischung von 200 μl eingehalten wurde.
%Hemmung bei einer festen Konzentration wurden wie folgt errechnet:

$$(1\text{-Enzymaktivität}_{\text{gehemmte Reaktion}} / \text{Enzymaktivität}_{\text{ungehemmte Reaktion}}) \times 100$$

Tabelle 2: Biologische Aktivität

| Ausführungsbeispiel Nr. | % Hemmung bei 30 μM |
|---|---|
| 2 | 8 |
| 3 | 20 |

Aus der Tabelle ist abzulesen, dass die Verbindungen der Formel I die Aktivität der DPP-IV (Dipeptidyl Peptidase IV) hemmen und dadurch zur Senkung des Blutzuckerspiegels geeignet sind.

Nachfolgend wird die Herstellung einiger Beispiele detailliert beschrieben, die anderen Beispiele wurden analog erhalten:

Beispiel 1

N-[2-(3-Amino-propylsulfanyl)-1-(3-methyl-but-2-enyl)-1H-benzimidazol-6-yl]-benzamid Trifluoressigsäuresalz

a) [3-(5/6-Nitro-1H-benzimidazol-2-ylsulfanyl)-propyl]-carbaminsäure-tert-butylester

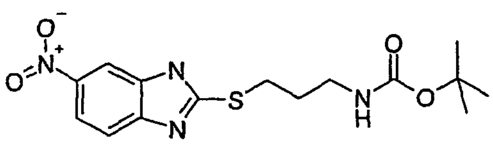

+

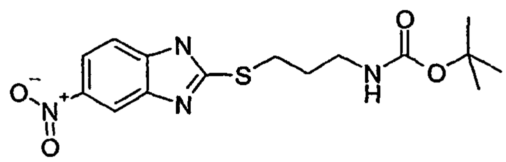

Eine Lösung von 1,0 g (5,12 mmol) 5-Nitro-benzimidazol-2-thiol und 790 µl (5,63 mmol) Triethylamin in 40 ml Tetrahydrofuran wurde 10 Minuten gerührt und anschließend mit einer Lösung von (3-Brom-propyl)-carbaminsäure-tert-butylester in 10 ml Tetrahydrofuran versetzt. Es wurde auf 70˚C erhitzt und 72 Stunden gerührt. Das Reaktionsgemisch wurde im Vakuum eingeengt und mittels präparativer HPLC (Acetonitril/Wasser + 0,5 % Trifluoressigsäure, Gradient: 20/80 auf 100/0) getrennt. Es wurden 180 mg (18%) Edukt und 1,17 g (65%) des gewünschten Produktes erhalten.
MS: m/z = 353 $(M+H)^+$.

b) {3-[1-(3-Methyl-but-2-enyl)-5-nitro-1H-benzimidazol-2-ylsulfanyl]-propyl}-carbaminsäure-tert-butylester

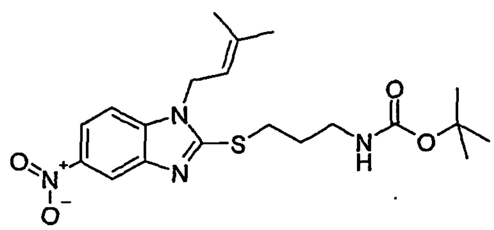

Zu einer Lösung von 1,15 g (3,26 mmol) [3-(5/6-Nitro-1H-benzimidazol-2-ylsulfanyl)-propyl]-carbaminsäure-tert-butylester in 20 ml Dimethylformamid gab man 1,60 g (4,90 mmol) Cäsiumkarbonat und rührte 30 Minuten bei Raumtemperatur. Anschließend wurden 535 mg (3,59 mmol) 1-Bromo-3-methyl-2-buten langsam zugetropft. Das Reaktionsgemisch wurde im Vakuum eingeengt und zwischen Wasser und Dichlormethan verteilt. Die organische Phase wurde getrocknet und im Vakuum eingeengt. Das Rohgemisch wurde an Kieselgel (Heptan/Essigsäureethylester, Gradient: 90:10 auf 0:100) getrennt. Man erhielt 145 mg (11%) des gewünschten Produktes, 426 mg (31%) einer Mischfraktion und 406 mg (30%) {3-[1-(3-Methyl-but-2-enyl)-5-nitro-1H-benzimidazol-2-ylsulfanyl]-propyl}-carbaminsäure-tert-butylester.
MS : m/z = 421 $(M+H)^+$.

c) {3-[6-Amino-1-(3-methyl-but-2-enyl)-1H-benzimidazol-2-ylsulfanyl]-propyl}-carbaminsäure-tert-butylester

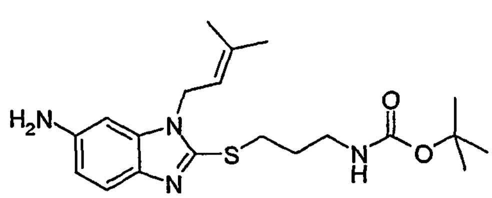

Zu einer Suspension von 93 mg (1,66 mmol) Eisen und 16 mg (0,30 mmol) Ammoniumchlorid in 1,5 ml Wasser wurde eine Lösung von 140 mg (0,33 mmol) {3-[1-(3-Methyl-but-2-enyl)-6-nitro-1H-benzimidazol-2-ylsulfanyl]-propyl}-carbaminsäure-tert-butylester in 15 ml Ethanol getropft und 3 Stunden bei Rückfluss gekocht. Der Katalysator wurde abfiltriert und mit Ethanol gewaschen. Das Filtrat wurde im Vakuum eingeengt. Die so erhaltenen 138 mg des gewünschten Produktes wurden ohne weitere Reinigung in der nächsten Stufe eingesetzt.

d) {3-[6-Benzoylamino-1-(3-methyl-but-2-enyl)-1H-benzimidazol-2-ylsulfanyl]-propyl}-carbaminsäure-tert-butylester

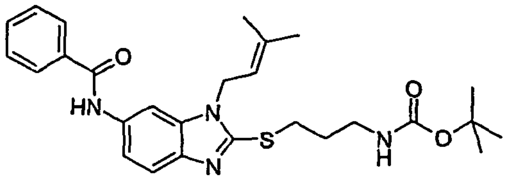

Zu einer Lösung von 58 mg {3-[6-Amino-1-(3-methyl-but-2-enyl)-1H-benzimidazol-2-ylsulfanyl]-propyl}-carbaminsäure-tert-butylester (analog Beipsiel la-c erhalten) in 5 ml Dimethylformamid gab man 24 mg (0,07 mmol) Cäsiumkarbonat und rührte 30 Minuten bei Raumtemperatur. Anschließend wurden 17 µl (0,15 mmol) Benzoylbromid zugegeben und 20 Stunden bei Raumtemperatur gerührt. Das Reaktionsgemisch wurde im Vakuum eingeengt und zwischen Wasser und Dichlormethan verteilt. Die organische Phase wurde getrocknet, im Vakuum eingeengt und mittels präparativer HPLC (Acetonitril/Wasser + 0,5% Trifluoressigsäure, Gradient: 20/80 auf 100/0) gereinigt. Man erhielt 33 mg (44%) des gewünschten Produktes.
MS: m/z = 495 $(M+H)^+$.

e) N-[2-(3-Amino-propylsulfanyl)-1-(3-methyl-but-2-enyl)-1H-benzimidazol-6-yl]-benzamid Trifluoressigsäuresalz (A003423930A)

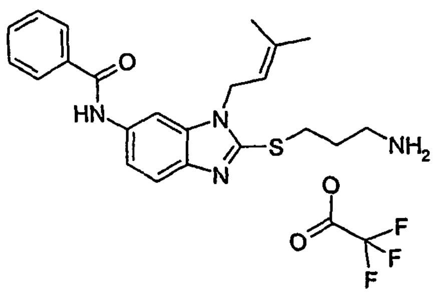

33 mg (0,07 mmol) {3-[6-Benzoylamino-1-(3-methyl-but-2-enyl)-1H-benzimidazol-2-ylsulfanyl]-propyl}-carbaminsäure-tert-butylester wurden in 102 µl Trifluoressigsäure und 100 µl Wasser gelöst und 6 Stunden bei Raumtemperatur gerührt. Das Reaktionsgemisch wurde mit Wasser verdünnt und gefriergetrocknet. Man erhielt 34 mg des gewünschten Produktes in quantitativer Ausbeute.
MS: m/z = 509 $(M+H)^+$.

## Patentansprüche

1. Verbindungen der Formel I,

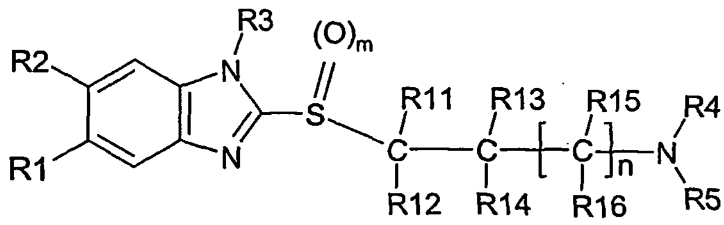

I

worin bedeuten

R1, R2 unabhängig voneinander H, CONR20R21, NR22COR23 oder NR24R25, wobei nicht beide Reste R1 oder R2 Wasserstoff entsprechen können;

R20 ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl oder ($C_1$-$C_6$)-Alkylen- ($C_6$-$C_{10}$)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alk-yl, O-($C_1$-$C_6$)-Alkyl, S- ($C_1$-$C_6$)-Alkyl, S(O)-($C_1$-$C_6$)-Alkyl oder $S(O)_2$-($C_1$-$C_6$)-Alkyl;

R21 H, ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1C_6$)-Alkylen-($C_6$-$C_{10}$)-Aryl oder ($C_1$-$C_6$)-Alkylen- ($C_6$-$C_{10}$)-Heterocyclyl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_5$)-Alkyl, S- ($C_1$-$C_6$)-Alkyl, S(O)-($C_1$-$C_6$)-Alkyl oder $S(O)_2$-($C_1$-$C_6$)-Alkyl;

R22, R23 unabhängig voneinander H, ($C_1$-$C_{10}$)-Alkyl, ($C_3$-$C_{10}$)-Cycloalkyl, ($C_2$-$C_{10}$)- Alkenyl, ($C_2$-$C_{10}$)-Alkinyl, ($C_6$-$C_{10}$)-Aryl, Heterocyclyl, ($C_1$-$C_6$)-Alkylen-($C_6$- $C_{10}$)-Aryl, ($C_1$-$C_6$)-Alkylen-($C_6$-$C_{10}$)-Heterocyclyl oder S $(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Alkyl oder $S(O)_2$-($C_1$-$C_6$)-Alkyl;

R24 ($C_1$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl, ($C_1$-$C_6$)-Alkylen-($C_6$- $C_{10}$)-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Alkyl oder S $(O)_2$-($C_1$-$C_6$)-Alkyl;

R25 H, ($C_1$-$C_{10}$)-Alkyl, ($C_3$-$C_{10}$)-Cyloalkyl, ($C_2$-$C_{10}$)-Alkenyl, ($C_2$-$C_{10}$)-Alkinyl, ($C_6$- $C_{10}$)-Aryl, Heterocyclyl, ($C_1$-$C_6$)-Alkylen-($C_6$-$C_{10}$)-Aryl, ($C_1$-$C_6$)-Alkylen-($C_6$- $C_{10}$)-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, ($C_1$-$C_6$)-Alkyl, O-($C_1$-$C_6$)-Alkyl, S-($C_1$-$C_6$)-Alkyl, S(O)-($C_1$-$C_6$)-Alkyl oder S $(O)_2$-($C_1$-$C_6$)-Alkyl;

R3 $CF_3$, ($C_2$-$C_{10}$)-Alkyl, ($C_3$-$C_{10}$)-Cycloalkyl, ($C_2$-$C_{10}$)-Alkenyl, ($C_2$-$C_{10}$)-Alkinyl, ($C_6$-$C_{10}$)-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, ($C_1$-$C_6$)-Alkyl, -$CF_3$, -$OCF_3$, -$SCF_3$, ($C_2$-$C_6$)- Alkenyl, ($C_2$-$C_6$)-Alkinyl, OR7, $OP(O)(OR7)_2$, NR7R8, NR7CONR7R8, COR7, OCOR7, OCOOR7, COOR7, CONR7R8, OCONR7R8, ($C_1$-$C_6$)-Alkylen-OR7, ($C_1$-$C_6$)-Alkylen-NR7R8, ($C_1$-$C_6$)-Alkylen-NR7S$(O)_2$R7, ($C_1$-$C_6$)-Alkylen-SR7, ($C_1$-$C_6$)-Alkylen-S(O)R7, ($C_1$-$C_6$)-Alkylen-S$(O)_2$R7, ($C_1$-$C_6$)-Alkylen- S$(O)_2$NR7R8, ($C_1$-$C_6$)-Alkylen-COR7, ($C_1$-$C_6$)-Alkylen-COOR7, ($C_1$-$C_6$)- Alkylen-CONR7R8, SR7, S(O)R7, S$(O)_2$R7, S$(O)_2$NR7R8, NR7S $(O)_2$R7, ($C_1$- $C_6$)-Alkylen-($C_3$-$C_{10}$)-Cycloalkyl, ($C_1$-$C_6$)-Alkylen-($C_6$-$C_{10}$)-Aryl, ($C_1$-$C_6$)- Alkylen-Heterocyclyl, ($C_3$-$C_{10}$)-Cycloalkyl, ($C_6$-$C_{10}$)-Aryl oder Heterocyclyl;

wobei die Bedeutung unsubstituiertes oder substituiertes Piperidin-4-yl und unsubstituiertes oder substituiertes -$CH_2$-($C_6H_4$)-($C_6H_5$) ausgenommen sind.

R7, R8 unabhängig voneinander H, ($C_1$-$C_6$)-Alkyl, -$CF_3$, ($C_3$-$C_{10}$)-Cycloalkyl, ($C_6$-$C_{10}$)- Aryl, Heterocyclyl, ($C_1$-$C_6$)-Alkylen-CONR9R10, CONR9R10, ($C_1$-$C_6$)-Alkylen- COOR9, COOR9, COR9, ($C_1$-$C_6$)-Alkylen-COR9, ($C_1$-$C_6$)-Alkylen-OR9, ($C_1$- $C_6$)-Alkylen-NR9R10, ($C_1$-$C_6$)-Alkylen-SR9, ($C_1$-$C_6$)-Alkylen-S(O)R9, ($C_1$-$C_6$)- Akylen-S$(O)_2$R9, S(O)R9, S$(O)_2$R9, ($C_1$-$C_4$)-Alkylen-($C_6$-$C_{10}$)-Aryl oder ($C_1$- $C_4$)-Alkylen-Heterocyclyl;

R9, R10 unabhängig voneinander H, $(C_1-C_6)$-Alkyl, $(C_1-C_6)$-Alkylen-$(C_6-C_{10})$-Aryl, -$(C_6- C_{10})$-Aryl, Heterocyclyl oder $(C_1-C_6)$-Alkylen-Heterocyclyl;
R4, R5 unabhängig voneinander H, $(C_1-C_6)$-Allcyl oder $(C_3-C_8)$-Cykloalkyl, wobei $(C_1- C_6)$-Alkyl oder $(C_3-C_8)$-Cykloalkyl substitiuert sein können mit F, Cl, Br, I, CN, Aryl, Heterocyclyl, $NH_2$, $NH(C_1-C_6)$-Alkyl, $N((C_1-C_6)$-Alkyl$)_2$, OH, $O(C_1-C_6)$- Alkyl, OAryl, OHeteroaryl, $S(C_1-C_6)$-Alkyl, $S(O)(C_1-C_6)$-Alkyl oder $S(O)_2(C_1- C_6)$-Alkyl, wobei diese Alkylgruppen wiederum mit F, Cl, Br oder I substituiert sein können;
R11, R12, R13, R14, R15, R16 unabhängig voneinander H, $(C_1-C_6)$-Akyl, Aryl, Heterocyclyl, $(C_3-C_8)$-Cykloalkyl, $(C_1-C_4)$-Alkylen-O-$(C_1-C_6)$-Alkyl,$(C_1-C_4)$-AlkylenS-$(C_1-C_6)$-Alkyl,$(C_1-C_4)$-Alkylen-NH-$(C_1-C_6)$-Akyl,$(C_1-C_4)$-Alkylen- N(Alkyl$)_2$, $(C_1-C_4)$-Alkylen-$(C_6-C_{10})$-Aryl, $(C_1-C_4)$-Alkylen-Heterocyclyl, F, Cl, Br, I, CN, COOH, COO $(C_1-C_6)$-Akyl, $CONH_2$, $CONH(C_1-C_6)$-Alkyl, $CON((C_1- C_6)$-Alkyl$)_2$, $CF_3$,
oder zwei der Reste R11, R12, R13, R14, R15, R16 bilden gemeinsam einen $(C_2-C_6)$- Alkylenrest, an dem ein $(C_6-C_{10})$-Arylrest oder ein $(C_6-C_{10})$-Heterocyclylrest ankondensiert sein kann und diese Arylreste oder Heterocyclylreste mit F, Cl, Br, I, $OCF_3$, $CF_3$, CN, $(C_1-C_6)$-Alkyl, Aryl, Heterocyclyl, $(C_3-C_8)$-Cykloalkyl, $(C_1- C_4)$-Alkylen-O-$(C_1-C_6)$-Alkyl, $(C_1-C_4)$-Alkylen-S-$(C_1-C_6)$-Alkyl, $(C_1-C_4)$- Alkylen-NH-$(C_1-C_6)$-Alkyl, $(C_1-C_4)$-Alkylen-$N((C_1-C_6)$-Alkyl$)_2$, $(C_1-C_4)$- Alkylen-$(C_6-C_{10})$-Aryl, $(C_1-C_4)$-Alkylen-Heterocyclyl, COOH, $COO(C_1-C_6)$- Alkyl, $CONH_2$, $CONH(C_1-C_6)$-Alkyl, $CON((C_1-C_6)$-Alkyl$)_2$, OH, O-$(C_1-C_6)$- Alkyl, O-$(C_3-C_6)$-Cycloalkyl, S-$(C_1-C_6)$-Alkyl, S-$(C_3-C_6)$-Cycloalkyl, SO-$(C_1- C_6)$-Alkyl, SO-$(C_3-C_6)$-Cycloalkyl, $SO_2$-$(C_1-C_6)$-Alkyl, $SO_2$-$(C_3-C_6)$-Cycloalkyl, $SO_2$-$NH_2$, $SO_2$-NH-$(C_1-C_6)$-Alkyl oder $SO_2$-NH-$(C_3-C_7)$-Cycloalkyl substituiert sein können;
m 0, 1, oder 2;
n 0 oder 1;

sowie deren physiologisch verträglichen Salze.

**2.** Verbindungen der Formel I, gemäß Anspruch 1, **dadurch gekennzeichnet, dass** darin bedeuten

R1, R2 unabhängig voneinander H, NR22COR23 oder NR24R25, wobei nicht beide Reste R1 oder R2 Wasserstoff entsprechen können;
R22, R23 unabhängig voneinander H, $(C_1-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, $(C_1-C_6)$-Alkylen-$(C_6- C_{10})$-Aryl, $(C_1-C_6)$-Alkylen-$(C_6-C_{10})$-Heterocyclyl oder S $(O)_2$-Aryl, wobei die Alkyl-, Cycloalkyl-, Alkenyl,-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder $S(O)_2$-$(C_1-C_6)$-Alkyl;
R24 $(C_1-C_{10})$-Alkyl, $(C_3-C_{10})$-Cycloalkyl, $(C_2-C_{10})$-Alkenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder S $(O)_2$-$(C_1-C_6)$-Alkyl;
R25 H, $(C_1-C_{10})$-Alkyl, $(C_3-C_{10})$-Cycloalkyl, $(C_2-C_{10})$-Alkenyl, $(C_2-C_{10})$-Alkinyl, $(C_6$- Cio)-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Akyl, S-$(C_1-C_6)$-Alkyl, S(O)-$(C_1-C_6)$-Akyl oder S $(O)_2$-$(C_1-C_6)$-Alkyl;
R3 $CF_3$, $(C_2-C_{10})$-Alkyl, $(C_3-C_{10})$-Cycloalkyl, $(C_2-C_{10})$-Alkenyl, $(C_2-C_{10})$-Alkinyl, $(C_6-C_{10})$-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, $(C_1-C_6)$-Alkyl, -$CF_3$, -$OCF_3$, -$SCF_3$, $(C_2-C_6)$- Alkenyl, $(C_2-C_6)$-Alkinyl, OR7, $OP(O)(OR7)_2$, NR7R8, NR7CONR7R8, COR7, OCOR7, OCOOR7, COOR7, CONR7R8, OCONR7R8, $(C_1-C_6)$-Alkylen-OR7, $(C_1-C_6)$-Alkylen-NR7R8, $(C_1-C_6)$-Alkylen-NR7$S(O)_2$R7, $(C_1-C_6)$-Alkylen-SR7, $(C_1-C_6)$-Alkylen-S(O)R7, $(C_1-C_6)$-Alkylen-$S(O)_2$R7, $(C_1-C_6)$-Alkylen- $S(O)_2$NR7R8, $(C_1-C_6)$-Alkylen-COR7, $(C_1-C_6)$-Alkylen-COOR7, $(C_1-C_6)$- Alkylen-CONR7R8, SR7, S(O)R7, $S(O)_2$R7, $S(O)_2$NR7R8, NR7S $(O)_2$R7, $(C_1- C_6)$-Alkylen-$(C_3-C_{10})$-Cycloalkyl, $(C_1$-C6)-Alkylen-$(C_6-C_{10})$-Aryl, $(C_1-C_6)$- Alkylen-Heterocyclyl, $(C_3-C_{10})$-Cycloalkyl, $(C_6-C_{10})$-Aryl oder Heterocyclyl;

wobei die Bedeutung unsubstituiertes oder substituiertes Piperidin-4-yl und unsubstituiertes oder substituiertes -$CH_2$-$(C_6H_4)$-$(C_6H_5)$ ausgenommen sind.

R7, R8 unabhängig voneinander H, $(C_1-C_6)$-Alkyl, -$CF_3$, $(C_3-C_{10})$-Cycloalkyl, $(C_6-C_{10})$- Aryl, Heterocyclyl, $(C_1-C_6)$-Alkylen-CONR9R10, CONR9R10, $(C_1-C_6)$-Alkylen- COOR9, COOR9, COR9, $(C_1-C_6)$-Alkylen-COR9, $(C_1-C_6)$-Alkylen-OR9, $(C_1- C_6)$-Alkylen-NR9R10, $(C_1-C_6)$-Alkylen-SR9, $(C_1-C_6)$-Alkylen-S(O)R9, $(C_1-C_6)$- Al-

kylen-$S(O)_2R9$, S(O)R9, $S(O)_2R9$, $(C_1-C_4)$-Alkylen-$(C_6-C_{10})$-Aryl oder $(C_1- C_4)$-Alkylen-Heterocyclyl;
R9, R10 unabhängig voneinander H, $(C_1-C_6)$-Alkyl, $(C_1-C_6)$-Alkylen-$(C_6-C_{10})$-Aryl, -$(C_6- C_{10})$-Aryl, Heterocyclyl oder $(C_1-C_6)$-Alkylen-Heterocyclyl;
$R_{4,}$ R5 unabhängig voneinander H, $(C_1-C_6)$-Alkyl oder $(C_3-C_8)$-Cykloalkyl, wobei $(C_1- C_6)$-Alkyl oder $(C_3-C_8)$-Cykloalkyl substitiuert sein können mit F, Cl, Br, I, CN, Aryl, Heterocyclyl, $NH_2$, $NH(C_1-C_6)$-Alkyl, $N((C_1-C_6)\text{-Alkyl})_2$, OH, $O(C_1-C_6)$- Alkyl, OAryl, OHeteroaryl, $S(C_1-C_6)$-Alkyl, $S(O)(C_1-C_6)$-Alkyl oder $S(O)_2(C_1- C_6)$-Alkyl, wobei diese Alkylgruppen wiederum mit F, Cl, Br oder I substituiert sein können;
R11, R12, R13, R14, R15, R16 unabhängig voneinander H, $(C_1-C_6)$-Akyl, Aryl, Heterocyclyl, $(C_3-C_8)$-Cykloalkyl, $(C_1-C_4)$-Alkylen-O-$(C_1-C_6)$-Alkyl, $(C_1-C_4)$- Alkylen-S-$(C_1-C_6)$-Alkyl, $(C_1-C_4)$-Alkylen-NH-$(C_1-C_6)$-Alkyl, $(C_1-C_4)$-Alkylen- $N(Alkyl)_2$, $(C_1-C_4)$-Alkylen-$(C_6-C_{10})$-Aryl, $(C_1-C_4)$-Alkylen-Heterocyclyl, F, Cl, Br, I, CN, COOH, $COO(C_1-C_6)$-Alkyl, $CONH_2$, $CONH(C_1-C_6)$-Alkyl, $CON((C_1- C_6)\text{-Alkyl})_2$, $CF_3$,
oder zwei der Reste R11, R12, R13, R14, R15, R16 bilden gemeinsam einen $(C_2-C_6)$- Alkylenrest, an dem ein $(C_6-C_{10})$-Arylrest oder ein $(C_6-C_{10})$-Heterocyclylrest ankondensiert sein kann und diese Arylreste oder Heterocyclylreste mit F, Cl, Br, I, $OCF_3$, $CF_3$, CN, $(C_1-C_6)$-Alkyl, Aryl, Heterocyclyl, $(C_3-C_8)$-Cykloalkyl, $(C_1- C_4)$-Alkylen-O-$(C_1-C_6)$-Alkyl, $(C_1-C_4)$-Alkylen-S-$(C_1-C_6)$-Alkyl, $(C_1-C_4)$- Alkylen-NH-$(C_1-C_6)$-Alkyl, $(C_1-C_4)$-Akylen-$N((C_1-C_6)\text{-Alkyl})_2$, $(C_1-C_4)$- Alkylen-$(C_6-C_{10})$-Aryl, $(C_1-C_4)$-Alkylen-Heterocyclyl, COOH, $COO(C_1-C_6)$- Alkyl, $CONH_2$, $CONH(C_1-C_6)$-Alkyl, $CON((C_1-C_6)\text{-Alkyl})_2$, OH, O-$(C_1-C_6)$- Alkyl, O-$(C_3-C_6)$-Cycloalkyl, S-$(C_1-C_6)$-Alkyl, S-$(C_3-C_6)$-Cycloalkyl, SO-$(C_1- C_6)$-Alkyl, SO-$(C_3-C_6)$-Cycloalkyl, $SO_2$-$(C_1-C_6)$-Alkyl, $SO_2$-$(C_3-C_6)$-Cycloalkyl, $SO_2$-$NH_2$, $SO_2$-NH-$(C_1-C_6)$-Alkyl oder $SO_2$-NH-$(C_3-C_7)$-Cycloalkyl substituiert sein können;
m 0, 1, oder 2;
n 0 oder 1;

sowie deren physiologisch verträglichen Salze.

3. Verbindungen der Formel I, gemäß Anspruch 1 oder 2, **dadurch gekennzeichnet, dass** darin bedeuten

R1 H;
R2 NR22COR23 oder NR24R25;
R22, R23 unabhängig voneinander H, $(C_1-C_{10})$-Alkyl, $(C_3-C_{10})$-Cycloalkyl, $(C_2-C_{10})$- Alkenyl, $(C_2-C_{10})$-Alkinyl, $(C_6-C_{10})$-Aryl, Heterocyclyl, $(C_1-C_6)$-Alkylen-$(C_6- C_{10})$-Aryl, $(C_1-C_6)$-Alkylen-$(C_6-C_{10})$-Heterocyclyl oder S $(O)_2$-Aryl, wobei die Alkyl-, Cycloalkyl-, Alkenyl-, Alkinyl-, Alkylen-, Aryl- und Heterocyclyl-Reste ein oder mehrfach substituiert sein können mit F, Cl, Br, I, CN, $NO_2$, SH, OH, $(C_1-C_6)$-Alkyl, O-$(C_1-C_6)$-Akyl, S-$(C_1-C_6)$-Alkyl, S(O)-$(C_1-C_6)$-Alkyl oder $S(O)_2$-$(C_1-C_6)$-Alkyl;
R24 $(C_1-C_{10})$-Alkyl, $(C_3-C_{10})$-Cycloalkyl, $(C_2-C_{10}$-Alkenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-$(C_6- C_{10})$-Heterocyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder S $(O)_2$-$(C_1-C_6)$-Alkyl;
R25 H, $(C_1-C_{10})$-Alkyl, $(C_3-C_{10})$-Cycloalkyl, $(C_2-C_{10})$-Alkenyl, $(C_2-C_{10})$-Alkinyl, $(C_6- C_{10})$-Aryl, Heterocyclyl, $(C_1-C_6)$-Alkylen-$(C_6-C_{10})$-Aryl, $(C_1-C_6)$-Alkylen-(C6- $C_{10})$-Heteroeyclyl oder $S(O)_2$-Aryl, wobei die Alkyl-, Cycloalkyl-, Alkenyl-, Alkinyl-, Alkylen-, Aryl- und Heterocyclyl-Reste ein- oder mehrfach substituiert sein können mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder S $(O)_2$-$(C_1-C_6)$-Alkyl;
$R^3$ $CF^3$, $(C_2-C_{10})$-Alkyl, $(C_3-C_{30})$-Cyclocloalkyl, $(C_2-C_{10})$-Alkenyl, $(C_2-C_{10})$-Alkinyl, $(C_6-C_{10})$-Aryl, Heterocyclyl, wobei die Alkyl-, Cycloalkyl-, Alkenyl-, Alkinyl-, Aryl- und Heterocyclyl Reste ein- oder mehrfach substituiert sein können mit F, Cl, Br, I, CN, $NO_2$, SH, OH, $(C_1-C_6)$-Alkyl, $-CF_3$, $-OCF_3$, $-SCF_3$, $(C_2-C_6)$- Alkenyl, $(C_2-C_6)$-Alkinyl, $OR^7$, $OP(O)(OR7)_2$, NR7R8, NR7CONR7R8, COR7, OCOR7, OCOOR7, COOR7, CONR7R8, OCONR7R8, $(C_1-C_6)$-Alkylen-OR7, $(C_1-C_6)$-Alkylen-NR7R8, $(C_1-C_6)$-Alkylen-$NR7S(O)_2R7$, $(C_1-C_6)$-Alkylen-SR7, $(C_1-C_6)$-Alkylen-S(O)R7, $(C_1-C_6)$-Alkylen-$S(O)_2R7$, $(C_1-C_6)$-Alkylen- $S(O)_2NR7R8$, $(C_1-C_6)$-Alkylen-COR7, $(C_1-C_6)$-Alkylen-COOR7, $(C_1-C_6)$- Alkylen-CONR7R8, SR7, S(O)R7, $S(O)_2R7$, $S(O)_2NR7R8$, NR7S $(O)_2R7$, $(C_1- C_6)$-Alkylen-$(C_3-C_{10})$-Cycloalkyl, $(C_1-C_6)$-Akylen-$(C_6-C_{10})$-Aryl, $(C_1-C_6)$- Alkylen-Heterocyclyl, $(C_3-C_{10})$-Cycloalkyl, $(C_6-C_{10})$-Aryl oder Heterocyclyl;

wobei die Bedeutung unsubstituiertes oder substituiertes Piperidin-4-yl und unsubstituiertes oder substituiertes $-CH_2-(C_6H_4)-(C_6H_5)$ ausgenommen sind.

R7, R8 unabhängig voneinander H, $(C_1-C_6)$-Alkyl, $-CF_3$, $(C_3-C_{10})$-Cycloalkyl, $(C_6-C_{10})$- Aryl, Heterocyclyl, $(C_1-C_6)$-Alkylen-CONR9R10, CONR9R10, $(C_1-C_6)$-Alkylen- COOR9, COOR9, COR9, $(C_1-C_6)$-Alkylen-COR9,

$(C_1-C_6)$-Alkylen-OR9, $(C_1-C_6)$-Alkylen-NR9R10, $(C_1-C_6)$-Alkylen-SR9, $(C_1-C_6)$-Alkylen-S(O)R9, $(C_1-C_6)$-Alkylen-$S(O)_2$R9, S(O)R9, $S(O)_2$R9, $(C_1-C_4)$-Alkylen-$(C_6-C_{10})$-Aryl oder $(C_1-C_4)$-Alkylen-Heterocyclyl;
R9, R10 unabhängig voneinander H, $(C_1-C_6)$-Alkyl, $(C_1-C_6)$-Alkylen-$(C_6-C_{10})$-Aryl, -$(C_6-C_{10})$-Aryl, Heterocyclyl oder $(C_1-C_6)$-Alkylen-Heterocyclyl;
R4, R5 unabhängig voneinander H, $(C_1-C_6)$-Alkyl oder $(C_3-C_8)$-Cykloalkyl, wobei $(C_1-C_6)$-Alkyl oder $(C_3-C_8)$-Cykloalkyl substitiuert sein können mit F, Cl, Br, I, CN, Aryl, Heterocyclyl, $NH_2$, $NH(C_1-C_6)$-Alkyl, $N((C_1-C_6)\text{-Alkyl})_2$, OH, $O(C_1-C_6)$- Alkyl, OAryl, OHeteroaryl, $S(C_1-C_6)$-Alkyl, $S(O)(C_1-C_6)$-Alkyl oder $S(O)_2(C_1-C_6)$-Alkyl, wobei diese Alkylgruppen wiederum mit F, Cl, Br oder I substituiert sein können;
R11, R12, R13, R14, R15, R16 unabhängig voneinander H, $(C_1-C_6)$-Alkyl, Aryl, Heterocyclyl, $(C_3-C_8)$-Cykloalkyl, $(C_1-C_4)$-Akylen-O-$(C_1-C_6)$-Akyl,$(C_1-C_4)$-Alkylen-S-$(C_1-C_6)$-Alkyl,$(C_1-C_4)$-Alkylen-NH-$(C_1-C_6)$-Alkyl,$(C_1-C_4)$-Alkylen- $N(Alkyl)_2$, $(C_1-C_4)$-Alkylen-$(C_6-C_{10})$-Aryl, $(C_1-C_4)$-Alkylen-Heterocyclyl, F, Cl, Br, I, CN, COOH, COO $(C_1-C_6)$-Alkyl, $CONH_2$, $CONH(C_1-C_6)$-Alkyl, $CON((C_1-C_6)\text{-Alkyl})_2$, $CF_3$,
oder zwei der Reste R11, R12, R13, R14, R15, R16 bilden gemeinsam einen $(C_2-C_6)$- Alkylenrest, an dem ein $(C_6-C_{10})$-Arylrest oder ein $(C_6-C_{10})$-Heterocyclylrest ankondensiert sein kann und diese Arylreste oder Heterocyclylreste mit F, Cl, Br, I, $OCF_3$, $CF_3$, CN, $(C_1-C_6)$-Alkyl, Aryl, Heterocyclyl, $(C_3-C_8)$-Cykloalkyl, $(C_1-C_4)$-Alkylen-O-$(C_1-C_6)$-Alkyl, $(C_1-C_4)$-Alkylen-S-$(C_1-C_6)$-Alkyl, $(C_1-C_4)$- Alkylen-NH-$(C_1-C_6)$-Alkyl, $(C_1-C_4)$-Alkylen-$N((C_1-C_6)\text{-Alkyl})_2$, $(C_1-C_4)$- Alkylen-$(C_6-C_{10})$-Aryl, $(C_1-C_4)$-Alkylen-Heterocyclyl, COOH, $COO(C_1-C_6)$- Alkyl, $CONH_2$, $CONH(C_1-C_6)$-Alkyl, $CON((C_1-C_6)\text{-Alkyl})_2$, OH, O-$(C_1-C_6)$- Alkyl, O-$(C_3-C_6)$-Cycloalkyl, S-$(C_1-C_6)$-Alkyl, S-$(C_3-C_6)$-Cycloalkyl, SO-$(C_1-C_6)$-Alkyl, SO-$(C_3-C_6)$-Cycloalkyl, $SO_2$-$(C_1-C_6)$-Alkyl, $SO_2$-$(C_3-C_6)$-Cycloalkyl, $SO_2$-$NH_2$, $SO_2$-NH-$(C_1-C_6)$-Alkyl oder $SO_2$-NH-$(C_3-C_7)$-Cycloalkyl substituiert sein können;
m 0;
n 0 oder 1;

sowie deren physiologisch verträglichen Salze.

**4.** Verbindungen der Formel I, gemäß einem oder mehreren der Ansprüche 1 bis 3 , **dadurch gekennzeichnet, dass** darin bedeuten

R1 H;
R2 NR22COR23 oder NR24R25;
R22 H;
R23 $(C_6-C_{10})$-Aryl, wobei der Aryl-Rest ein- oder mehrfach substituiert sein kann mit F, Cl, Br, I, CN, $NO_2$, SH, OH, $(C_1-C_6)$-Alkyl, O-$(C_1-C_6)$-Alkyl, S-$(C_1-C_6)$- Alkyl, S(O)-$(C_1-C_6)$-Alkyl oder $S(O)_2$-$(C_1-C_6)$-Alkyl;
R24 $(C_1-C_6)$-Alkylen-$(C_6-C_{10})$-Aryl, wobei der Aryl-Rest ein- oder mehrfach substituiert sein kann mit F, Cl, Br, I, CN, $NO_2$, SH, OH, $(C_1-C_6)$-Alkyl, O-$(C_1-C_6)$-Alkyl, S-$(C_1-C_6)$-Alkyl, S(O)-$(C_1-C_6)$-Alkyl oder $S(O)_2$-$(C_1-C_6)$-Alkyl;
R25 H;
R3 $(C_2-C_{10})$-Alkenyl
R4, R5 H;
R11, R12, R13, R14, R15, R16 H;
n 0 oder 1;

sowie deren physiologisch verträglichen Salze.

**5.** Verbindungen gemäß einem oder mehreren der Ansprüche 1 bis 4 zur Anwendung als Arzneimittel.

**6.** Arzneimittel enthaltend eine oder mehrere der Verbindungen gemäß einem oder mehreren der Ansprüche 1 bis 4.

**7.** Arzneimittel enthaltend eine oder mehrere der Verbindungen gemäß einem oder mehreren der Ansprüche 1 bis 4 und mindestens einen weiteren Wirkstoff.

**8.** Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alfa Agonisten, PPAR alfa/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulfonylharnstoffe, Biguanide, Meglitinide, Thiazolidindione, $\alpha$-Glukosidase-Inhibitoren, auf den ATP-abhängigen Kaliumkanal der Betazellen wirkende Wirkstoffe,

CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

**9.** Verwendung der Verbindungen gemäß einem oder mehreren der Ansprüche 1 bis 4 zur Herstellung eines Medikamentes zur Blutzuckersenkung.

**10.** Verwendung der Verbindungen gemäß einem oder mehreren der Ansprüche 1 bis 4 zur Herstellung eines Medikamentes zur Behandlung des Typ II Diabetes.

**11.** Verwendung der Verbindungen gemäß einem oder mehreren der Ansprüche 1 bis 4 zur Herstellung eines Medikamentes zur Behandlung von Lipid- und Kohlenhydratstoffwechselstörungen.

**12.** Verwendung der Verbindungen gemäß einem oder mehreren der Ansprüche 1 bis 4 zur Herstellung eines Medikaments zur Behandlung arteriosklerotischer Erscheinungen.

**13.** Verwendung der Verbindungen gemäß einem oder mehreren der Ansprüche 1 bis 4 zur Herstellung eines Medikaments zur Behandlung von Insulin Resistenz.

**14.** Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

## Claims

**1.** A compound of the formula I

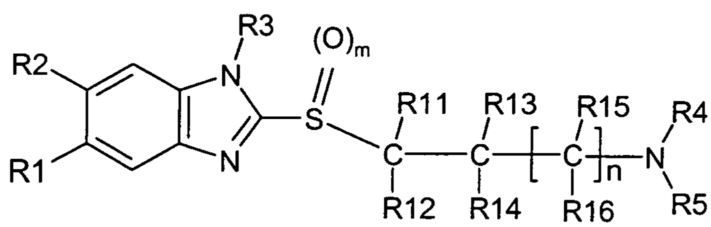

I

in which

R1, R2 are each independently H, CONR20R21, NR22COR23 or NR24R25, where the two R1 and R2 radicals cannot both be hydrogen;
R20 is ($C_1$-$C_{10}$) -alkyl, ($C_3$-$C_{10}$) -cycloalkyl, ($C_2$-$C_{10}$)- alkenyl, ($C_2$-$C_{10}$) -alkynyl, ($C_6$-$C_{10}$)-aryl, heterocyclyl, ($C_1$-$C_6$) -alkylene- ($C_6$-$C_{10}$) -aryl or ($C_1$-$C_6$) -alkylene- ($C_6$-$C_{10}$) -heterocyclyl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)- alkyl, S-($C_1$-$C_6$)-alkyl, S(O)-($C_1$-$C_6$)-alkyl or $S(O)_2$-($C_1$-$C_6$)-alkyl;
R21 is H, ($C_1$-$C_{10}$) -alkyl, ($C_3$-$C_{10}$) -cycloalkyl, ($C_2$-$C_{10}$)- alkenyl, ($C_2$-$C_{10}$)-alkynyl, ($C_6$-$C_{10}$)-aryl, heterocyclyl, ($C_1$-$C_6$) -alkylene- ($C_6$-$C_{10}$)-aryl or ($C_1$-$C_6$)-alkylene- ($C_6$-$C_{10}$)-heterocyclyl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)- alkyl, S-($C_1$-$C_6$)-alkyl, S(O)-($C_1$-$C_6$)-alkyl or $S(O)_2$-($C_1$-$C_6$)-alkyl;
R22, R23 are each independently H, ($C_1$-$C_{10}$)-alkyl, ($C_3$- $C_{10}$)-cycloalkyl, ($C_2$-$C_{10}$)-alkenyl, ($C_2$-$C_{10}$)- alkynyl, ($C_6$-$C_{10}$)-aryl, heterocyclyl, ($C_1$-$C_6$)- alkylene-($C_6$-$C_{10}$) -aryl, ($C_1$-$C_6$)-alkylene- ($C_6$- $C_{10}$)-heterocyclyl or $S(O)_2$-ar-

yl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)- alkyl, S-($C_1$-$C_6$)-alkyl , S(O)-($C_1$-$C_6$)-alkyl or $S(O)_2$-($C_1$-$C_6$)-alkyl;

R24 is ($C_1$-$C_{10}$)-alkyl, ($C_3$-$C_{10}$)-cycloalkyl, ($C_2$-$C_{10}$)- alkenyl, ($C_2$-$C_{10}$)-alkynyl, ($C_6$-$C_{10}$)-aryl, heterocyclyl, ($C_1$-$C_6$) -alkylene- ($C_6$-$C_{10}$)-aryl, ($C_1$-$C_6$) -alkylene- ($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl , O-($C_1$-$C_6$)-alkyl, S-($C_1$-$C_6$)- alkyl, S(O)-($C_1$-$C_6$)-alkyl or $S(O)_2$-($C_1$-$C_6$)- alkyl;

R25 is H, ($C_1$-$C_{10}$) -alkyl, ($C_3$-$C_{10}$) -cycloalkyl, ($C_2$-$C_{10}$)- alkenyl, ($C_2$-$C_{10}$) -alkynyl, ($C_6$-$C_{10}$) -aryl, heterocyclyl, ($C_1$-$C_6$)-alkylene-($C_6$-$C_{10}$)-aryl, ($C_1$-$C_6$) -alkylene- ($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)-alkyl, S-($C_1$-$C_6$)- alkyl, S(O)-($C_1$-$C_6$) -alkyl or $S(O)_2$-($C_1$-$C_6$)- alkyl;

R3 is $CF_3$, ($C_2$-$C_{10}$) -alkyl, ($C_3$-$C_{10}$) -cycloalkyl, ($C_2$-$C_{10}$)- alkenyl, ($C_2$-$C_{10}$) -alkynyl, ($C_6$-$C_{10}$)-aryl, heterocyclyl, where the alkyl, cycloalkyl, alkenyl, alkynyl, aryl and heterocyclyl radicals may be mono- or polysubstituted by F, Cl, Br, I, CN, $NO_2$, SH, OH, ($C_1$-$C_6$)-alkyl, -$CF_3$, -$OCF_3$, -$SCF_3$, ($C_2$-$C_6$)-alkenyl, ($C_2$-$C_6$)- alkynyl, OR7, OP(O)$(OR7)_2$, NR7R8, NR7CONR7R8, COR7, OCOR7, OCOOR7, COOR7, CONR7R8, OCONR7R8, ($C_1$-$C_6$)-alkylene-OR7, ($C_1$-$C_6$)- alkylene-NR7R8, ($C_1$-$C_6$)-alkylene-NR7$S(O)_2$R7, ($C_1$-$C_6$) -alkylene-SR7, (C1-C6)-alkylene-S(O)R7, ($C_1$-$C_6$)-alkylene-$S(O)_2$R7, ($C_1$-$C_6$)-alkylene- $S(O)_2$NR7R8, (C1-C6)-alkylene-COR7, ($C_1$-$C_6$)- alkylene-COOR7, ($C_1$-$C_6$)-alkylene-CONR7R8, SR7, S(O)R7, $S(O)_2$R7, $S(O)_2$NR7R8, NR7$S(O)_2$R7, ($C_1$- $C_6$)-alkylene- ($C_3$-$C_{10}$)-cycloalkyl, ($C_1$-$C_6$)- alkylene- ($C_6$-$C_{10}$) -aryl, ($C_1$-$C_6$)-alkylene- heterocyclyl, ($C_3$-$C_{10}$) -cycloalkyl, ($C_6$-$C_{10}$)- aryl or heterocyclyl;

excluding the definition of unsubstituted or substituted piperidin-4-yl and unsubstituted or substituted -$CH_2$-($C_6H_4$)-($C_6H_5$).

R7, R8 are each independently H, ($C_1$-$C_6$)-alkyl, -$CF_3$, ($C_3$-$C_{10}$)-cycloalkyl, ($C_6$-$C_{10}$)-aryl, heterocyclyl, ($C_1$-$C_6$)-alkylene-CONR9R10, CONR9R10, ($C_1$-$C_6$)-alkylene-COOR9, COOR9, COR9, ($C_1$-$C_6$)-alkylene-COR9, ($C_1$-$C_6$)-alkylene-OR9, ($C_1$-$C_6$)-alkylene-NR9R10, ($C_1$-$C_6$)-alkylene-SR9, ($C_1$-$C_6$)-alkylene-S(O)R9, ($C_1$-$C_6$)-alkylene- $S(O)_2$R9, S(O)R9, $S(O)_2$R9, ($C_1$-$C_4$)-alkylene-($C_6$- $C_{10}$)-aryl or ($C_1$-$C_4$)-alkylene-heterocyclyl;

R9, R10 are each independently H, ($C_1$-$C_6$)-alkyl, ($C_1$-$C_6$)- alkylene- ($C_6$-$C_{10}$)-aryl, - ($C_6$-$C_{10}$)-aryl, heterocyclyl or ($C_1$-$C_6$)-alkylene-heterocyclyl;

R4, R5 are each independently H, ($C_1$-$C_6$)-alkyl or ($C_3$- $C_8$)-cycloalkyl, where ($C_1$-$C_6$)-alkyl or ($C_3$-$C_8$)- cycloalkyl may be substituted by F, Cl, Br, I, CN, aryl, heterocyclyl, $NH_2$, NH($C_1$-$C_6$)-alkyl, N(($C_1$-$C_6$)-alkyl$)_2$, OH, O($C_1$-$C_6$)-alkyl, Oaryl, Oheteroaryl, S($C_1$-$C_6$)-alkyl, S(O)($C_1$-$C_6$)-alkyl or $S(O)_2$($C_1$-$C_6$)-alkyl, where these alkyl groups may in turn be substituted by F, Cl, Br or I;

R11, R12, R13, R14, R15, R16 are each independently H, ($C_1$-$C_6$)-alkyl, aryl, heterocyclyl, ($C_3$-$C_8$)- cycloalkyl, ($C_1$-$C_4$)-alkylene-O-($C_1$-$C_6$)-alkyl, ($C_1$-$C_4$)-alkylene-S-($C_1$-$C_6$)-alkyl, ($C_1$-$C_4$)- alkylene-NH-($C_1$-$C_6$)-alkyl, ($C_1$-$C_4$)-alkylene- N(alkyl$)_2$, ($C_1$-$C_4$)-alkylene-($C_6$-$C_{10}$)-aryl, ($C_1$- $C_4$)-alkyleneheterocyclyl, F, Cl, Br, I, CN, COOH, COO($C_1$-$C_6$)-alkyl, $CONH_2$, CONH($C_1$-$C_6$)- alkyl, CON(($C_1$-$C_6$)-alkyl$)_2$, $CF_3$,

or two of the R11, R12, R13, R14, R15, R16 radicals together form a ($C_2$-$C_6$)-alkylene radical to which may be fused a ($C_6$-$C_{10}$)-aryl radical or a ($C_6$-$C_{10}$)-heterocyclyl radical, and these aryl or heterocyclyl radicals may be substituted by F, Cl, Br, I, $OCF_3$, $CF_3$, CN, ($C_1$-$C_6$)-alkyl, aryl, heterocyclyl, ($C_3$-$C_8$)-cycloalkyl, ($C_1$- $C_4$) -alkylene-O- ($C_1$-$C_6$) -alkyl, ($C_1$-$C_4$) -alkylene- S-($C_1$-$C_6$)-alkyl, ($C_1$-$C_4$) -alkylene-NH- ($C_1$-$C_6$)- alkyl, ($C_1$-C4)-alkylene-N(($C_1$-$C_6$)-alkyl$)_2$, ($C_1$- $C_4$) -alkylene- ($C_6$-$C_{10}$)-aryl, ($C_1$-$C_4$)- alkyleneheterocyclyl, COOH, COO($C_1$-$C_6$)-alkyl, $CONH_2$, CONH($C_1$-$C_6$)-alkyl, CON(($C_1$-$C_6$)-alkyl$)_2$, OH, O-($C_1$-$C_6$)-alkyl, O-($C_3$-$C_6$)-cycloalkyl, S- ($C_1$-$C_6$)-alkyl, S-($C_3$-$C_6$)-cycloalkyl, SO-($C_1$-$C_6$)- alkyl, SO-($C_3$-$C_6$)-cycloalkyl, $SO_2$-($C_1$-$C_6$)- alkyl, $SO_2$-($C_3$-$C_6$)-cycloalkyl, $SO_2$-$NH_2$, $SO_2$-NH- ($C_1$-$C_6$)-alkyl or $SO_2$-NH-($C_3$-$C_7$)-cycloalkyl;

m is 0, 1 or 2;

n is 0 or 1;

and the physiologically compatible salts thereof.

**2.** A compound of the formula I as claimed in claim 1, wherein

R1, R2 are each independently H, NR22COR23 or NR24R25, where the two R1 and R2 radicals cannot both be hydrogen;

R22, R23 are each independently H, ($C_1$-$C_{10}$)-alkyl, ($C_3$- $C_{10}$)-cycloalkyl, ($C_2$-$C_{10}$)-alkenyl, ($C_2$-$C_{10}$)- alkynyl, ($C_6$-$C_{10}$)-aryl, heterocyclyl, ($C_1$-$C_6$)- alkylene- ($C_6$-$C_{10}$)-aryl, ($C_1$-$C_6$)-alkylene- ($C_6$- $C_{10}$)-heterocyclyl or $S(O)_2$-ar-

yl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)- alkyl, S-($C_1$-$C_6$)-alkyl, S(O)-($C_1$-$C_6$)-alkyl or $S(O)_2$-($C_1$-$C_6$)-alkyl;

R24 is ($C_1$-$C_{10}$)-alkyl, ($C_3$-$C_{10}$)-cycloalkyl, ($C_2$-$C_{10}$)- alkenyl, ($C_2$-$C_{10}$)-alkynyl, ($C_6$-$C_{10}$)-aryl, heterocyclyl, ($C_1$-$C_6$)-alkylene-($C_6$-$C_{10}$)-aryl, ($C_1$-$C_6$)-alkylene-($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$) alkyl, O-($C_1$-$C_6$)-alkyl, S-($C_1$-$C_6$)- alkyl, S(O)-($C_1$-$C_6$)-alkyl or $S(O)_2$-($C_1$-$C_6$)- alkyl;

R25 is H, ($C_1$-$C_{10}$)-alkyl, ($C_3$-$C_{10}$)-cycloalkyl, ($C_2$-$C_{10}$)- alkenyl, ($C_2$-$C_{10}$)-alkynyl, ($C_6$-$C_{10}$)-aryl, heterocyclyl, ($C_1$-$C_6$)-alkylene-($C_6$-$C_{10}$)-aryl, ($C_1$-$C_6$) -alkylene-($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)-alkyl, S-($C_1$-$C_6$)- alkyl, S(O)-($C_1$-$C_6$)-alkyl or $S(O)_2$-($C_1$-$C_6$)- alkyl;

R3 is $CF_3$, ($C_2$-$C_{10}$)-alkyl, ($C_3$-$C_{10}$)-cycloalkyl, ($C_2$-$C_{10}$)- alkenyl, ($C_2$-$C_{10}$) -alkynyl, ($C_6$-$C_{10}$) -aryl, heterocyclyl, where the alkyl, cycloalkyl, alkenyl, alkynyl, aryl and heterocyclyl radicals may be mono- or polysubstituted by F, Cl, Br, I, CN, $NO_2$, SH, OH, ($C_1$-$C_6$)-alkyl, -$CF_3$, -$OCF_3$, -$SCF_3$, ($C_2$-$C_6$)-alkenyl, ($C_2$-$C_6$)- alkynyl, OR7, OP(O)($OR7)_2$, NR7R8, NR7CONR7R8, COR7, OCOR7, OCOOR7, COOR7, CONR7R8, OCONR7R8, ($C_1$-$C_6$)-alkylene-OR7, ($C_1$-$C_6$)- alkylene-NR7R8, ($C_1$-$C_6$)-alkylene-NR7$S(O)_2$R7, ($C_1$-$C_6$)-alkylene-SR7, ($C_1$-$C_6$)-alkylene-S(O)R7, ($C_1$-$C_6$)-alkylene-$S(O)_2$R7, ($C_1$-$C_6$)-alkylene- $S(O)_2$NR7R8, ($C_1$-$C_6$)-alkylene-COR7, ($C_1$-$C_6$)- alkylene-COOR7, ($C_1$-$C_6$)-alkylene-CONR7R8, SR7, S(O)R7, $S(O)_2$R7, $S(O)_2$NR7R8, NR7$S(O)_2$R7, ($C_1$- $C_6$)-alkylene-(C3-$C_{10}$)-cycloalkyl, ($C_1$-$C_6$)- alkylene-($C_6$-$C_{10}$)-aryl, ($C_1$-$C_6$)-alkylene- heterocyclyl, ($C_3$-$C_{10}$) -cycloalkyl, ($C_6$-$C_{10}$) aryl or heterocyclyl;

excluding the definition of unsubstituted or substituted piperidin-4-yl and unsubstituted or substituted -$CH_2$-($C_6H_4$)-($C_6H_5$).

R7, R8 are each independently H, ($C_1$-$C_6$)-alkyl , -$CF_3$, ($C_3$-$C_{10}$) -cycloalkyl, ($C_6$-$C_{10}$)-aryl, heterocyclyl, ($C_1$-$C_6$)-alkylene-CONR9R10, CONR9R10, ($C_1$-$C_6$)-alkylene-COOR9, COOR9, COR9, ($C_1$-$C_6$)-alkylene-COR9, ($C_1$-$C_6$)-alkylene-OR9, ($C_1$-$C_6$)-alkylene-NR9R10, ($C_1$-$C_6$) -alkylene-SR9, ($C_1$-$C_6$)-alkylene-S(O)R9, ($C_1$-$C_6$)-alkylene- $S(O)_2$R9, S(O)R9, $S(O)_2$R9, ($C_1$-$C_4$)-alkylene- ($C_6$- $C_{10}$)-aryl or ($C_1$-$C_4$)-alkylene-heterocyclyl;

R9, R10 are each independently H, ($C_1$-$C_6$)-alkyl, ($C_1$-$C_6$)- alkylene-($C_6$-$C_{10}$)-aryl, -($C_6$-$C_{10}$)-aryl, heterocyclyl or ($C_1$-$C_6$)-alkylene-heterocyclyl;

R4, R5 are each independently H, ($C_1$-$C_6$)-alkyl or ($C_3$- $C_8$) -cycloalkyl, where ($C_1$-$C_6$)-alkyl or ($C_3$-$C_8$)- cycloalkyl may be substituted by F, Cl, Br, I, CN, aryl, heterocyclyl, $NH_2$, NH($C_1$-$C_6$)-alkyl, N(($C_1$-$C_6$)-alkyl$)_2$, OH, O($C_1$-$C_6$)-alkyl, Oaryl, Oheteroaryl, S($C_1$-$C_6$)-alkyl, S(O)($C_1$-$C_6$)-alkyl or $S(O)_2$($C_1$-$C_6$)-alkyl, where these alkyl groups may in turn be substituted by F, Cl, Br or I;

R11, R12, R13, R14, R15, R16 are each independently H, ($C_1$-$C_6$)-alkyl, aryl, heterocyclyl, ($C_3$-$C_8$)- cycloalkyl, ($C_1$-$C_4$) -alkylene-O-($C_1$-$C_6$)-alkyl, ($C_1$-$C_4$)-alkylene-S-($C_1$-$C_6$)-alkyl, ($C_1$-$C_4$)- alkylene-NH- ($C_1$-$C_6$)-alkyl, ($C_1$-$C_4$)-alkylene- N(alkyl$)_2$, ($C_1$-$C_4$)-alkylen-($C_6$-$C_{10}$)-aryl, ($C_1$- $C_4$)-alkyleneheterocyclyl, F, Cl, Br, I, CN, COOH, COO ($C_1$-$C_6$)-alkyl, $CONH_2$, CONH($C_1$-$C_6$)- alkyl, CON (($C_1$-$C_6$)-alkyl$)_2$, $CF_3$,

or two of the R11, R12, R13, R14, R15, R16 radicals together form a ($C_2$-$C_6$)-alkylene radical to which may be fused a ($C_6$-$C_{10}$)-aryl radical or a ($C_6$-$C_{10}$)-heterocyclyl radical, and these aryl or heterocyclyl radicals may be substituted by F, Cl, Br, I, $OCF_3$, $CF_3$, CN, ($C_1$-$C_6$)-alkyl, aryl, heterocyclyl, ($C_3$-$C_8$)-cycloalkyl, ($C_1$- $C_4$)-alkylene-O-($C_1$-$C_6$)-alkyl, ($C_1$-$C_4$)-alkylene- S-($C_1$-$C_6$)-alkyl, ($C_1$-$C_4$)-alkylene-NH- ($C_1$-$C_6$)- alkyl, ($C_1$-$C_4$)-alkylene-N(($C_1$-$C_6$)-alkyl$)_2$, ($C_1$- $C_4$) -alkylene- ($C_6$-$C_{10}$) -aryl, ($C_1$-$C_4$)- alkyleneheterocyclyl, COOH, COO($C_1$-$C_6$)-alkyl, $CONH_2$, CONH ($C_1$-$C_6$)-alkyl, CON(($C_1$-$C_6$)-alkyl$)_2$, OH, O-($C_1$-$C_6$)-alkyl, O-($C_3$-$C_6$)-cycloalkyl, S- ($C_1$-$C_6$)-alkyl, S-($C_3$-$C_6$)-cycloalkyl, SO-($C_1$-$C_6$)- alkyl, SO-($C_3$-$C_6$)-cycloalkyl, $SO_2$-($C_1$-$C_6$)- alkyl, $SO_2$-($C_3$-$C_6$)-cycloalkyl, $SO_2$-$NH_2$, $SO_2$-NH- ($C_1$-$C_6$)-alkyl or $SO_2$-NH-($C_3$-$C_7$)-cycloalkyl;

m is 0, 1 or 2;

n is 0 or 1;

and the physiologically compatible salts thereof.

**3.** A compound of the formula I as claimed in claim 1 or 2, wherein

R1 is H;

R2 is NR22COR23 or NR24R25;

R22, R23 are each independently H, ($C_1$-$C_{10}$)-alkyl, ($C_3$- $C_{10}$)-cycloalkyl, ($C_2$-$C_{10}$)-alkenyl, ($C_2$-$C_{10}$)- alkynyl, ($C_6$-$C_{10}$)-aryl, heterocyclyl, ($C_1$-$C_6$)- alkylene- ($C_6$-$C_{10}$)-aryl, ($C_1$-$C_6$)-alkylene- ($C_6$- $C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)- alkyl, S-($C_1$-$C_6$)-alkyl, S(O)-($C_1$-$C_6$)-alkyl or $S(O)_2$-($C_1$-$C_6$)-alkyl;

R24 is ($C_1$-$C_{10}$)-alkyl, ($C_3$-$C_{10}$)-cycloalkyl, ($C_2$-$C_{10}$)- alkenyl, ($C_2$-$C_{10}$)-alkynyl, ($C_6$-$C_{10}$)-aryl, heterocyclyl, ($C_1$-$C_6$)-alkylene-($C_6$-$C_{10}$)-aryl, ($C_1$-$C_6$)-alkylene-($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)-alkyl, S-($C_1$-$C_6$)- alkyl, S(O)-($C_1$-$C_6$)-alkyl or $S(O)_2$-($C_1$-$C_6$)- alkyl;

R25 is H, ($C_1$-$C_{10}$)-alkyl, ($C_3$-$C_{10}$)-cycloalkyl, ($C_2$-$C_{10}$)- alkenyl, ($C_2$-$C_{10}$) -alkynyl, ($C_6$-$C_{10}$)-aryl, heterocyclyl, ($C_1$-$C_6$) -alkylene- ($C_6$-$C_{10}$) -aryl, ($C_1$-$C_6$) -alkylene- ($C_6$-$C_{10}$)-heterocyclyl or $S(O)_2$-aryl, where the alkyl, cycloalkyl, alkenyl, alkynyl, alkylene, aryl and heterocyclyl radicals may be mono- or polysubstituted by F, Cl, Br, I, CN, $NO_2$, SH, OH, ($C_1$-$C_6$)-alkyl, O-($C_1$-$C_6$)-alkyl, S-($C_1$-$C_6$)- alkyl, S(O)-($C_1$-$C_6$)-alkyl or $S(O)_2$-($C_1$-$C_6$)- alkyl;

R3 is $CF_3$, ($C_2$-$C_{10}$)-alkyl, ($C_3$-$C_{10}$)-cycloalkyl, ($C_2$-$C_{10}$)- alkenyl, ($C_2$-$C_{10}$)-alkynyl, ($C_6$-$C_{10}$)-aryl, heterocyclyl, where the alkyl, cycloalkyl, alkenyl, alkynyl, aryl and heterocyclyl radicals may be mono- or polysubstituted by F, Cl, Br, I, CN, $NO_2$, SH, OH, ($C_1$-$C_6$)-alkyl, -$CF_3$, -$OCF_3$, -$SCF_3$, ($C_2$-$C_6$)-alkenyl, ($C_2$-$C_6$)- alkynyl, OR7, OP(O)$(OR7)_2$, NR7R8, NR7CONR7R8, COR7, OCOR7, OCOOR7, COOR7, CONR7R8, OCONR7R8, ($C_1$-$C_6$)-alkylene-OR7, ($C_1$-$C_6$)- alkylene-NR7R8, ($C_1$-$C_6$)-alkylene-NR7$S(O)_2$R7, ($C_1$-$C_6$)-alkylene-SR7, ($C_1$-$C_6$)-alkylene-S(O)R7, ($C_1$-$C_6$)-alkylene-$S(O)_2$R7, ($C_1$-$C_6$)-alkylene- $S(O)_2$NR7R8, ($C_1$-$C_6$)-alkylene-COR7, ($C_1$-$C_6$)- alkylene-COOR7, ($C_1$-$C_6$)-alkylene-CONR7R8, SR7, S(O)R7, $S(O)_2$R7, $S(O)_2$NR7R8, NR7$S(O)_2$R7, ($C_1$- $C_6$)-alkylene-($C_3$-$C_{10}$)-cycloalkyl, ($C_1$-$C_6$)- alkylene- ($C_6$-$C_{10}$)-aryl, ($C_1$-$C_6$)-alkylene- heterocyclyl, ($C_3$-$C_{10}$)-cycloalkyl, ($C_6$-$C_{10}$)- aryl or heterocyclyl;

excluding the definition of unsubstituted or substituted piperidin-4-yl and unsubstituted or substituted -$CH_2$-($C_6H_4$)-($C_6H_5$).

R7, R8 are each independently H, ($C_1$-$C_6$)-alkyl, -$CF_3$, ($C_3$-$C_{10}$)-cycloalkyl, ($C_6$-$C_{10}$)-aryl, heterocyclyl, ($C_1$-$C_6$)-alkylene-CONR9R10, CONR9R10, ($C_1$-$C_6$)-alkylene-COOR9, COOR9, COR9, ($C_1$-$C_6$)-alkylene-COR9, ($C_1$-$C_6$)-alkylene-OR9, ($C_1$-$C_6$)-alkylene-NR9R10, ($C_1$-$C_6$)-alkylene-SR9, ($C_1$-$C_6$)-alkylene-S(O)R9, ($C_1$-$C_6$)-alkylene- $S(O)_2$R9, S(O)R9, S(O)2$R_9$, ($C_1$-$C_4$)-alkylene-($C_6$- $C_{10}$)-aryl or ($C_1$-$C_4$)-alkylene-heterocyclyl;

R9, R10 are each independently H, ($C_1$-$C_6$)-alkyl, ($C_1$-$C_6$)- alkylene- ($C_6$-$C_{10}$)-aryl, -($C_6$-$C_{10}$)-aryl, heterocyclyl or ($C_1$-$C_6$)-alkylene-heterocyclyl;

R4, R5 are each independently H, ($C_1$-$C_6$)-alkyl or ($C_3$- $C_8$)-cycloalkyl, where ($C_1$-$C_6$)-alkyl or ($C_3$-$C_8$)- cycloalkyl may be substituted by F, Cl, Br, I, CN, aryl, heterocyclyl, $NH_2$, NH($C_1$-$C_6$)-alkyl, N(($C_1$-$C_6$)-alkyl$)_2$, OH, O($C_1$-$C_6$)-alkyl, Oaryl, Oheteroaryl, S($C_1$-$C_6$)-alkyl, S(O)($C_1$-$C_6$)-alkyl or $S(O)_2$($C_1$-$C_6$)-alkyl, where these alkyl groups may in turn be substituted by F, Cl, Br or I;

R11, R12, R13, R14, R15, R16 are each independently H, ($C_1$-$C_6$)-alkyl, aryl, heterocyclyl, ($C_3$-$C_8$)- cycloalkyl, ($C_1$-$C_4$)-alkylene-O-($C_1$-$C_6$)-alkyl, ($C_1$-$C_4$)-alkylene-S-($C_1$-$C_6$)-alkyl, ($C_1$-$C_4$)- alkylene-NH-($C_1$-$C_6$)-alkyl, ($C_1$-$C_4$)-alkylene- N(alkyl$)_2$, ($C_1$-$C_4$)-alkylen-($C_6$-$C_{10}$)-aryl, ($C_1$- $C_4$)-alkyleneheterocyclyl, F, Cl, Br, I, CN, COOH, COO($C_1$-$C_6$)-alkyl, $CONCH_2$, CONH($C_1$-$C_6$)- alkyl, CON(($C_1$-$C_6$)-alkyl$)_2$, $CF_3$,

or two of the R11, R12, R13, R14, R15, R16 radicals together form a ($C_2$-$C_6$)-alkylene radical to which may be fused a ($C_6$-$C_{10}$)-aryl radical or a ($C_6$-$C_{10}$)-heterocyclyl radical, and these aryl or heterocyclyl radicals may be substituted by F, Cl, Br, I, $OCF_3$, $CF_3$, CN, ($C_1$-$C_6$)-alkyl, aryl, heterocyclyl, ($C_3$-$C_8$)-cycloalkyl, ($C_1$- $C_4$) -alkylene-O-($C_1$-$C_6$)-alkyl, ($C_1$-$C_4$)-alkylene- S-($C_1$-$C_6$)-alkyl, ($C_1$-$C_4$)-alkylene-NH-($C_1$-$C_6$)- alkyl, ($C_1$-$C_4$)-alkylene-N(($C_1$-$C_6$)-alkyl$)_2$, ($C_1$- $C_4$)-alkylene-($C_6$-$C_{10}$)-aryl, ($C_1$-$C_4$)- alkyleneheterocyclyl, COOH, COO($C_1$-$C_6$)-alkyl, $CONH_2$, CONH ($C_1$-$C_6$)-alkyl, CON (($C_1$-$C_6$)-alkyl$)_2$, OH, O-($C_1$-$C_6$)-alkyl, O-($C_3$-$C_6$)-cycloalkyl, S- ($C_1$-$C_6$)-alkyl, S-($C_3$-$C_6$)-cycloalkyl, SO-($C_1$-$C_6$)- alkyl, SO-($C_3$-$C_6$) -cycloalkyl, $SO_2$-($C_1$-$C_6$)- alkyl, $SO_2$-($C_3$-$C_6$)-cycloalkyl, $SO_2$-$NH_2$, $SO_2$-NH- ($C_1$-$C_6$)-alkyl or $SO_2$-NH-($C_3$-$C_7$)-cycloalkyl;

m is 0;

n is 0 or 1;

and the physiologically compatible salts thereof.

**4.** A compound of the formula I as claimed in one or more of claims 1 to 3, wherein

R1 is H;

R2 is NR22COR23 or NR24R25;

R22 is H;

R23 is ($C_6$-$C_{10}$)-aryl where the aryl radical may be mono- or polysubstituted by F, Cl, Br, I, CN, $NO_2$, SH, OH, ($C_1$-$C_6$)-alkyl, O-($C_1$-$C_6$)-alkyl, S- ($C_1$-$C_6$)-alkyl, S(O)-($C_1$-$C_6$)-alkyl or $S(O)_2$-($C_1$- $C_6$)-alkyl ;

R24 is ($C_1$-$C_6$)-alkylene-($C_6$-$C_{10}$)-aryl where the aryl radical may be mono- or polysubstituted by F, Cl, Br, I,

CN, $NO_2$, SH, OH, $(C_1-C_6)$-alkyl, O- $(C_1-C_6)$-alkyl, S-$(C_1-C_6)$-alkyl, S(O)-$(C_1-C_6)$- alkyl or $S(O)_2$-$(C_1-C_6)$-alkyl;
R25 is H;
R3 is $(C_2-C_{10})$-alkenyl
R4, R5 are each H;
R11, R12, R13, R14, R15, R16 are each H;
n is 0 or 1;

and the physiologically compatible salts thereof.

**5.** A compound as claimed in one or more of claims 1 to 4 for use as a medicament.

**6.** A medicament comprising one or more of the compounds as claimed in one or more of claims 1 to 4.

**7.** A medicament comprising one or more of the compounds as claimed in one or more of claims 1 to 4 and at least one further active ingredient.

**8.** A medicament as claimed in claim 7, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotonin and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptin, doprexin), lipase/amylase inhibitors, PPAR modulators, RXR modulators or TR-β agonists or amphetamines.

**9.** The use of the compounds as claimed in one or more of claims 1 to 4 for producing a medicament for lowering blood sugar.

**10.** The use of the compounds as claimed in one or more of claims 1 to 4 for producing a medicament for treating type II diabetes.

**11.** The use of the compounds as claimed in one or more of claims 1 to 4 for producing a medicament for treating lipid and carbohydrate metabolism disorders.

**12.** The use of the compounds as claimed in one or more of claims 1 to 4 for producing a medicament for treating arteriosclerotic manifestations.

**13.** The use of the compounds as claimed in one or more of claims 1 to 4 for producing a medicament for treating insulin resistance.

**14.** A process for producing a medicament comprising one or more of the compounds as claimed in one or more of claims 1 to 3, which comprises mixing the active ingredient with a pharmaceutically suitable carrier and bringing this mixture into a form suitable for administration.

## Revendications

**1.** Composés de formule 1

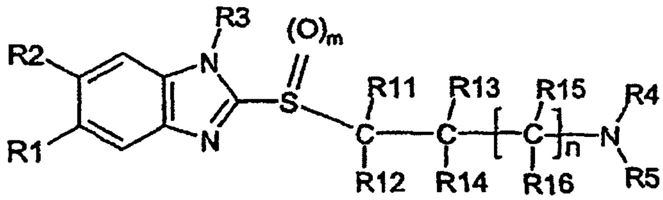

I

où

R1, R2 signifient, indépendamment l'un de l'autre, H, CONR20R21, NR22COR23 ou NR24R25, où les deux radicaux R1 ou R2 ne peuvent pas représenter hydrogène ;
R20 signifie ($C_1$-$C_{10}$)-alkyle, ($C_3$-$C_{10}$)-cycloalkyle, ($C_2$- $C_{10}$)-alcényle, ($C_2$-$C_{10}$)-alcynyle, ($C_6$-$C_{10}$)-aryle, hétérocyclyle, ($C_1$-$C_6$)-alkylène-($C_6$-$C_{10}$)-aryle ou ($C_1$-$C_6$)-alkylène- ($C_6$-$C_{10}$)-hétérocyclyle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)-alkyle, S-($C_1$-$C_6$)-alkyle, S(O)-($C_1$-$C_6$)-alkyle ou $S(O)_2$-($C_1$- $C_6$)-alkyle ;
R21 signifie H, ($C_1$-$C_{10}$)-alkyle, ($C_3$-$C_{10}$)-cycloalkyle, ($C_2$-$C_{10}$)-alcényle, ($C_2$-$C_{10}$)-alcynyle, ($C_6$-$C_{10}$)-aryle, hétérocyclyle, ($C_1$-$C_6$)-alkylène-($C_6$-$C_{10}$)-aryle ou ($C_1$-$C_6$)-alkylène- ($C_6$-$C_{10}$)-hétérocyclyle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)-alkyle, S-($C_1$-$C_6$)-alkyle, S(O)-($C_1$-$C_6$)-alkyle ou $S(O)_2$-($C_1$- $C_6$)-alkyle ;
R22, R23 signifient, indépendamment l'un de l'autre, H, ($C_1$-$C_{10}$)-alkyle, ($C_3$-$C_{10}$)-cycloalkyle, ($C_2$-$C_{10}$)- alcényle, ($C_2$-$C_{10}$)-alcynyle, ($C_6$-$C_{10}$)-aryle, hétérocyclyle, ($C_1$-$C_6$)-alkylène- ($C_6$-$C_{10}$)-aryle, ($C_1$- $C_6$)-alkylène-{$C_6$-$C_{10}$)-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S-($C_1$-$C_6$)-alkyle, S(O)-($C_1$-$C_6$)-alkyle ou $S(O)_2$-($C_1$-$C_6$)-alkyle ;
R24 signifie ($C_1$-$C_{10}$)-alkyle, ($C_3$-$C_{10}$)-cycloalkyle, ($C_2$- $C_{10}$)-alcényle, ($C_2$-$C_{10}$)-alcynyle, ($C_6$-$C_{10}$)-aryle, hétérocyclyle, ($C_1$-$C_6$)-alkylène-($C_6$-$C_{10}$)-aryle, ($C_1$- $C_6$)-alkylène- ($C_6$-$C_{10}$)-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S-($C_1$-$C_6$)-alkyle, S (O)-($C_1$-$C_6$)-alkyle ou $S(O)_2$-($C_1$-$C_6$)-alkyle ;
R25 signifie H, ($C_1$-$C_{10}$)-alkyle, ($C_3$-$C_{10}$)-cycloalkyle, ($C_2$-$C_{10}$) -alcényle, ($C_2$-$C_{10}$)-alcynyle, ($C_6$-$C_{10}$)-aryle, hétérocyclyle, ($C_1$-$C_6$}-alkylène-($C_6$-$C_{10}$)-aryle, ($C_1$- $C_6$)-alkylène-($C_6$-$C_{10}$)-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S- ($C_1$-$C_6$)-alkyle, S (O)-($C_1$-$C_6$)-alkyle ou S $(O)_2$-($C_1$-$C_6$)-alkyle ;
R3 signifie $CF_3$, ($C_2$-$C_{10}$)-alkyle, ($C_3$-$C_{10}$)-cycloalkyle, ($C_2$-$C_{10}$)-alcényle, ($C_2$-$C_{10}$)-alcynyle, ($C_6$-$C_{10}$)-aryle, hétérocyclyle, où les radicaux alkyle, cycloalkyle, alcényle, alcynyle, aryle et hétérocyclyle peuvent être monosubstitués ou polysubstitués par F, Cl, Br, I, CN, $NO_2$, SH, OH, ($C_1$-$C_6$)-alkyle, -$CF_3$, -$OCF_3$, -$SCF_3$, ($C_2$-$C_6$)-alcényle, ($C_2$-$C_6$)-alcynyle, OR7, OP(O) $(OR7)_2$, NR7R8, NR7CONR7R8, COR7, OCOR7, OCOOR7, COOR7, CONR7R8, OCONR7R8, ($C_1$-$C_6$)-alkylène-OR7, ($C_1$-$C_6$)-alkylène- NR7R8, ($C_1$-$C_6$)-alkylène-NR7$S(O)_2$R7, ($C_1$-$C_6$)- alkylène-SR7, ($C_1$-$C_6$)-alkylène-S(O)R7, ($C_1$-$C_6$)- alkylène-$S(O)_2$R7, ($C_1$-$C_6$)-alkylène-$S(O)_2$NR7R8, ($C_1$- $C_6$)-alkylène-COR7 , ($C_1$-$C_6$)-alkylène-COOR7, ($C_1$-$C_6$)- alkylène-CONR7R8, SR7, S(O)R7, $S(O)_2$R7, S $(O)_2$NR7R8,NR7$S(O)_2$R7,($C_1$-$C_6$)-alkylène-($C_3$-$C_{10}$)-cycloalkyle,($C_1$-$C_6$)-alkylène-($C_6$-$C_{10}$)-aryle,($C_1$-$C_6$)-alkylène- hétérocyclyle, ($C_3$-$C_{10}$)-cycloalkyle, ($C_6$-$C_{10}$)-aryle ou hétérocyclyle ;

où les significations pipéridin-4-yle non substitué ou substitué et -$CH_2$-($C_6H_4$)-($C_6H_5$) non substitué ou substitué sont exclues,

R7, R8 signifient, indépendamment l'un de l'autre, H, ($C_1$-$C_6$)-alkyle, -$CF_3$, ($C_3$-$C_{10}$)-cycloalkyle, ($C_6$-$C_{10}$)- aryle, hétérocyclyle, ($C_1$-$C_6$)-alkylène-CONR9R10, CONR9R10, ($C_1$-$C_6$)-alkylène-COOR9, COOR9, COR9, ($C_1$-$C_6$)-alkylène-COR9, ($C_1$-$C_6$)-alkylène-OR9, ($C_1$-$C_6$)- alkylène-NR9R10 , ($C_1$-$C_6$)-alkylène-SR9, ($C_1$-$C_6$)- alkylène-S(O)R9, ($C_1$-$C_6$)-alkylène-$S(O)_2$R9, S(O)R9, S(O)2$R_9$, ($C_1$-$C_4$)-alkylène- ($C_6$-$C_{10}$)-aryle ou ($C_1$-$C_4$)- alkylène-hétérocyclyle ;
R9, R10 signifient indépendamment l'un de l'autre H, ($C_1$-$C_6$)-alkyle, ($C_1$-$C_6$)-alkylène-($C_6$-$C_{10}$)-aryle,

-($C_6$-$C_{10}$)-aryle, hétérocyclyle ou ($C_1$-$C_6$)-alkylène- hétérocyclyle ;

R4, R5 signifient, indépendamment l'un de l'autre, H, ($C_1$-$C_6$)-alkyle ou ($C_3$-$C_8$)-cycloalkyle, où ($C_1$-$C_6$)- alkyle ou ($C_3$-$C_8$)-cycloalkyle peuvent être substitués par F, Cl, Br, I, CN, aryle, hétérocyclyle, $NH_2$, NH($C_1$-$C_6$)-alkyle, N(($C_1$-$C_6$)- alkyle)$_2$, OH, O($C_1$-$C_6$)-alkyle, O-aryle, O- hétéroaryle, S($C_1$-$C_6$)-alkyle, S(O) ($C_1$-$C_6$)-alkyle ou S (O)$_2$($C_1$-$C_6$)-alkyle, où ces groupes alkyle peuvent à leur tour être substitués par F, Cl, Br ou I ;

R11, R12, R13, R14, R15, R16 signifient, indépendamment l'un de l'autre, H, ($C_1$-$C_6$)-alkyle, aryle, hétérocyclyle, ($C_3$-$C_8$)-cycloalkyle, ($C_1$-$C_4$)- alkylène-O- ($C_1$-$C_6$)-alkyle, ($C_1$-$C_4$)-alkylène-S- ($C_1$- $C_6$) -alkyle, ($C_1$-$C_4$)-alkylène-NH-($C_1$-$C_6$)-alkyle, ($C_1$- $C_4$)-alkylène-N(alkyle)$_2$, ($C_1$-$C_4$)-alkylène-($C_6$-$C_{10}$)- aryle, ($C_1$-$C_4$)-alkylène-hétérocyclyle, F, Cl, Br, I, CN, COOH, COO($C_1$-$C_6$)-alkyle, $CONH_2$, CONH($C_1$-$C_6$)- alkyle, CON (($C_1$-$C_6$)-alkyle)$_2$, $CF_3$, ou deux des radicaux R11, R12, R13, R14, R15, R16 forment ensemble un radical ($C_2$-$C_6$)-alkylène, sur lequel peut être condensé un radical ($C_6$-$C_{10}$)-aryle ou un radical ($C_6$-$C_{10}$)hétérocyclyle et ces radicaux aryle ou hétérocyclyle peuvent être substitués par F, Cl, Br, I, $OCF_3$, $CF_3$, CN, ($C_1$-$C_6$)-alkyle, aryle, hétérocyclyle, ($C_3$-$C_8$)-cycloalkyle, ($C_1$-$C_4$)- alkylène-O-($C_1$-$C_6$)-alkyle, ($C_1$-$C_4$) -alkylène-S-($C_1$- $C_6$)-alkyle, ($C_1$-$C_4$)-alkylène-NH-($C_1$-$C_6$)-alkyle, ($C_1$- $C_4$)-alkylène-N(($C_1$-$C_6$)-alkyle)$_2$,($C_1$-$C_4$)-alkylène- ($C_6$-$C_{10}$)-aryle, ($C_1$-$C_4$)-alkylène-hétérocyclyle,COOH,COO($C_1$-$C_6$)-alkyle,$CONH_2$,CONH($C_1$-$C_6$)-alkyle,CON(($C_1$-$C_6$)-alkyle)$_2$,OH,O-($C_1$-$C_6$)-alkyle, O-($C_3$- $C_6$)-cycloalkyle, S-($C_1$-$C_6$)-alkyle, S-($C_3$-$C_6$)- cycloalkyle, SO-($C_1$-$C_6$)-alkyle, SO-($C_3$-$C_6$)- cycloalkyle, $SO_2$-(C1-$C_6$)-alkyle, $SO_2$-($C_3$-$C_6$)- cycloalkyle, $S0_2$-$NH_2$, $SO_2$-NH-($C_1$-$C_6$)-alkyle ou $SO_2$-NH-($C_3$-$C_7$)-cycloalkyle ;

m vaut 0, 1 ou 2 ;

n vaut 0 ou 1 ;

ainsi que leurs sels physiologiquement acceptables.

**2.** Composés de formule I, selon la revendication 1, **caractérisés en ce que**

R1, R2 signifient, indépendamment l'un de l'autre, H, NR22COR23 ou NR24R25, où les deux radicaux R1 ou R2 ne peuvent pas représenter hydrogène ;

R22, R23 signifient, indépendamment l'un de l'autre, H, ($C_1$-$C_{10}$)-alkyle, ($C_3$-$C_{10}$)-cycloalkyle, ($C_2$-$C_{10}$)- alcényle, ($C_2$-$C_{10}$)-alcynyle, ($C_6$-$C_{10}$)-aryle, hétérocyclyle, ($C_1$-$C_6$)-alkylène-($C_6$-$C_{10}$) -aryle, ($C_1$- $C_6$) -alkylène-($C_6$-$C_{10}$)-hétérocyclyle ou S(O)$_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S-($C_1$-$C_6$)-alkyle,S(O)-($C_1$-$C_6$)-alkyle ou S(O)$_2$-($C_1$-$C_6$)-alkyle ;

R24 signifie ($C_1$-$C_{10}$)-alkyle, ($C_3$-$C_{10}$)-cycloalkyle, ($C_2$- $C_{10}$)-alcényle, ($C_2$-$C_{10}$)-alcynyle, ($C_6$-$C_{10}$)-aryle, hétérocyclyle, ($C_1$-$C_6$) -alkylène- ($C_6$-$C_{10}$) -aryle, ($C_1$- $C_6$)-alkylène-($C_6$-$C_{10}$)-hétérocyclyle ou S(O)$_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S- ($C_1$-$C_6$)-alkyle, S (O)-($C_1$-$C_6$)-alkyle ou S(O)$_2$-($C_1$-$C_6$)-alkyle ;

R25 signifie H, ($C_1$-$C_{10}$)-alkyle, ($C_3$-$C_{10}$)-cycloalkyle, ($C_2$-$C_{10}$)-alcényle, ($C_2$-$C_{10}$)-alcynyle, ($C_6$-$C_{10}$)-aryle, hétérocyclyle, ($C_1$-$C_6$)-alkylène-($C_6$-$C_{10}$)-aryle, ($C_1$- $C_6$)-alkylène-($C_6$-$C_{10}$)-hétérocyclyle ou S(O)$_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, ($C_1$-$C_6$)-alkyle, O-($C_1$-$C_6$)- alkyle, S-($C_1$-$C_6$)-alkyle, S (O)-($C_1$-$C_6$)-alkyle ou S(O)$_2$-($C_1$-$C_6$)-alkyle ;

R3 signifie $CF_3$, ($C_2$-$C_{10}$)-alkyle, ($C_3$-$C_{10}$)-cycloalkyle, ($C_2$-$C_{10}$)-alcényle, ($C_2$-$C_{10}$)-alcynyle, ($C_6$-$C_{10}$)-aryle, hétérocyclyle, où les radicaux alkyle, cycloalkyle, alcényle, alcynyle, aryle et hétérocyclyle peuvent être monosubstitués ou polysubstitués par F, Cl, Br, I, CN, $NO_2$, SH, OH, ($C_1$-$C_6$)-alkyle, -$CF_3$, -$OCF_3$, -$SCF_3$, ($C_2$-$C_6$)-alcényle, ($C_2$-$C_6$)-alcynyle, OR7, OP(O)(OR7)$_2$, NR7R8, NR7CONR7R8, COR7, OCOR7, OCOOR7, COOR7, CONR7R8, OCONR7R8, ($C_1$-$C_6$)-alkylène-OR7, ($C_1$-$C_6$)-alkylène- NR7R8, ($C_1$-$C_6$)-alkylène-NR7S(O)$_2$R7, ($C_1$-$C_6$)- alkylène-SR7, ($C_1$-$C_6$)-alkylène-S(O)R7, ($C_1$-$C_6$)- alkylène-S(O)$_2$R7, ($C_1$-$C_6$)-alkylène-S(O)$_2$NR7R8, ($C_1$- $C_6$)-alkylène-COR7, ($C_1$-$C_6$)-alkylène-COOR7, ($C_1$-$C_6$)- alkylène-CONR7R8, SR7, S(O)R7, S(O)$_2$R7, S (O)$_2$NR7R8, NR7S(O)$_2$R7 , ($C_1$-$C_6$) -alkylène- ($C_3$-$C_{10}$)-cycloalkyle, ($C_1$-$C_6$)-alkylène- ($C_6$-$C_{10}$)-aryle, ($C_1$-$C_6$)-alkylène- hétérocyclyle, ($C_3$-$C_{10}$)-cycloalkyle, ($C_6$-$C_{10}$)-aryle ou hétérocyclyle ;

où les significations pipéridin-4-yle non substitué ou substitué et -$CH_2$-($C_6H_4$)-($C_6H_5$) non substitué ou substitué sont exclues,

R7, R8 signifient, indépendamment l'un de l'autre, H, ($C_1$-$C_6$)-alkyle, -$CF_3$, ($C_3$-$C_{10}$)-cycloalkyle, ($C_6$-$C_{10}$)- aryle, hétérocyclyle, ($C_1$-$C_6$)-alkylène-CONR9R10, CONR9R10, ($C_1$-$C_6$)-alkylène-COOR9, COOR9, COR9, ($C_1$-$C_6$)-alkylène-COR9, ($C_1$-$C_6$)-alkylène-OR9, ($C_1$-$C_6$)- alkylène-NR9R10, ($C_1$-$C_6$)-alkylène-SR9, ($C_1$-$C_6$)- alky-

lène-S(O)R9, $(C_1-C_6)$-alkylène-$S(O)_2$R9, S(O)R9, $S(O)_2R_9$, $(C_1-C_4)$-alkylène-$(C_6-C_{10})$-aryle ou $(C_1-C_4)$- alkylène-hétérocyclyle ;

R9, R10 signifient indépendamment l'un de l'autre H, $(C_1-C_6)$-alkyle, $(C_1-C_6)$-alkylène-$(C_6-C_{10})$-aryle, -$(C_6-C_{10})$-aryle, hétérocyclyle ou $(C_1-C_6)$-alkylène- hétérocyclyle ;

R4, R5 signifient, indépendamment l'un de l'autre, H, $(C_1-C_6)$ -alkyle ou $(C_3-C_8)$-cycloalkyle, où $(C_1-C_6)$- alkyle ou $(C_3-C_8)$-cycloalkyle peuvent être substitués par F, Cl, Br, I, CN, aryle, hétérocyclyle, $NH_2$, $NH(C_1-C_6)$-alkyle, N $((C_1-C_6)$- alkyle$)_2$, OH, $O(C_1-C_6)$-alkyle, O-aryle, O- hétéroaryle, $S(C_1-C_6)$-alkyle, $S(O)(C_1-C_6)$-alkyle ou S $(O)_2(C_1-C_6)$-alkyle, où ces groupes alkyle peuvent à leur tour être substitués par F, Cl, Br ou I ;

R11, R12, R13, R14, R15, R16 signifient, indépendamment l'un de l'autre, H, $(C_1-C_6)$-alkyle, aryle, hétérocyclyle, $(C_3-C_8)$-cycloalkyle, $(C_1-C_4)$- alkylène-O- $(C_1-C_6)$ -alkyle, $(C_1-C_4)$ -alkylène-S- $(C_1- C_6)$ -alkyle, $(C_1-C_4)$-alkylène-NH- $(C_1-C_6)$ -alkyle, $(C_1- C_4)$ -alkylène-N(alkyle$)_2$, $(C_1-C_4)$-alkylène-$(C_6-C_{10})$- aryle, $(C_1-C_4)$-alkylène-hétérocyclyle, F, Cl, Br, I, CN, COOH, $COO(C_1-C_6)$-alkyle, $CONH_2$, $CONH(C_1-C_6)$- alkyle, CON $((C_1-C_6)$-alkyle$)_2$, $CF_3$,

ou deux des radicaux R11, R12, R13, R14, R15, R16 forment ensemble un radical $(C_2-C_6)$-alkylène, sur lequel peut être condensé un radical $(C_6-C_{10})$-aryle ou un radical $(C_6-C_{10})$hétérocyclyle et ces radicaux aryle ou hétérocyclyle peuvent être substitués par F, Cl, Br, I, $OCF_3$, $CF_3$, CN, $(C_1-C_6)$-alkyle, aryle, hétérocyclyle, $(C_3-C_8)$-cycloalkyle, $(C_1-C_4)$- alkylène-O-$(C_1-C_6)$-alkyle, $(C_1-C_4)$-alkylène-S-$(C_1- C_6)$ -alkyle, $(C_1-C_4)$-alkylène-NH-$(C_1-C_6)$-alkyle, $(C_1- C_4)$-alkylène-N($(C_1-C_6)$-alkyle$)_2$, $(C_1-C_4)$-alkylène- $(C_6-C_{10})$-aryle, $(C_1-C_4)$-alkylène-hétérocyclyle, COOH, $COO(C_1-C_6)$-alkyle, $CONH_2$, $CONH(C_1-C_6)$-alkyle, CON($(C_1-C_6)$-alkyle$)_2$, OH, O-$(C_1-C_6)$-alkyle, O-$(C_3- C_6)$-cycloalkyle, S-$(C_1-C_6)$-alkyle, S-$(C_3-C_6)$- cycloalkyle, SO-$(C_1-C_6)$-alkyle, SO-$(C_3-C_6)$- cycloalkyle, $SO_2$-$(C_1-C_6)$-alkyle, $SO_2$-$(C_3-C_6)$- cycloalkyle, $SO_2$-$NH_2$, $SO_2$-NH-$(C_1-C_6)$-alkyle ou $SO_2$- NH-$(C_3-C_7)$-cycloalkyle ;

m vaut 0, 1 ou 2 ;

n vaut 0 ou 1 ;

ainsi que leurs sels physiologiquement acceptables.

**3.** Composés de formule I, selon la revendication 1 ou 2, **caractérisés en ce que**

R1 signifie H ;

R2 signifie NR22COR23 ou NR24R25 ;

R22, R23 signifient, indépendamment l'un de l'autre, H, $(C_1-C_{10})$-alkyle, $(C_3-C_{10})$-cycloalkyle, $(C_2-C_{10})$- alcényle, $(C_2-C_{10})$-alcynyle, $(C_6-C_{10})$-aryle, hétérocyclyle, $(C_1-C_6)$-alkylène-$(C_6-C_{10})$-aryle, $(C_1- C_6)$-alkylène-$(C_6-C_{10})$-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, $(C_1-C_6)$-alkyle, O-$(C_1-C_6)$- alkyle, S- $(C_1-C_6)$-alkyle, S (O)-$(C_1-C_6)$-alkyle ou $S(O)_2$-$(C_1-C_6)$-alkyle ;

R24 signifie $(C_1-C_{10})$-alkyle, $(C_3-C_{10})$-cycloalkyle, $(C_2- C_{10})$-alcényle, $(C_2-C_{10})$-alcynyle,$(C_6-C_{10})$-aryle, hétérocyclyle, $(C_1-C_6)$-alkylène-$(C_6-C_{10})$-aryle,$(C_1- C_6)$-alkylène-$(C_6-C_{10})$-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, $(C_1-C_6)$-alkyle, O-$(C_1-C_6)$- alkyle, S-$(C_1-C_6)$-alkyle,S (O)-$(C_1-C_6)$-alkyleou $S(O)_2$-$(C_1-C_6)$-alkyle ;

R25 signifie H, $(C_1-C_{10})$-alkyle, $(C_3-C_{10})$-cycloalkyle, $(C_2-C_{10})$-alcényle, $(C_2-C_{10})$-alcynyle, $(C_6-C_{10})$-aryle, hétérocyclyle, $(C_1-C_6)$-alkylène-$(C_6-C_{10})$-aryle,$(C_1- C_6)$-alkylène-$(C_6-C_{10})$-hétérocyclyle ou $S(O)_2$-aryle, où les radicaux alkyle, cycloalkyle, alcényle, alcynyle, alkylène, aryle et hétérocyclyle peuvent être monosubstitués ou polysubstitués par F, Cl, Br, I, CN, $NO_2$, SH, OH, $(C_1-C_6)$-alkyle, O-$(C_1-C_6)$- alkyle, S-$(C_1-C_6)$-alkyle, S (O)-$(C_1-C_6)$-alkyle ou $S(O)_2$-$(C_1-C_6)$-alkyle ;

R3 signifie $CF_3$, $(C_2-C_{10})$-alkyle, $(C_3-C_{10})$-cycloalkyle, $(C_2-C_{10})$-alcényle, $(C_2-C_{10})$-alcynyle, $(C_6-C_{10})$-aryle, hétérocyclyle, où les radicaux alkyle, cycloalkyle, alcényle, alcynyle, aryle et hétérocyclyle peuvent être monosubstitués ou polysubstitués par F, Cl, Br, I, CN, $NO_2$, SH, OH, $(C_1-C_6)$-alkyle, -$CF_3$, -$OCF_3$, -$SCF_3$, $(C_2-C_6)$-alcényle, $(C_2-C_6)$-alcynyle, OR7, OP(O)(OR7$)_2$, NR7R8, NR7CONR7R8, COR7, OCOR7, OCOOR7, COOR7, CONR7R8, OCONR7R8, $(C_1-C_6)$-alkylène-OR7, $(C_1-C_6)$-alkylène- NR7R8, $(C_1-C_6)$-alkylène-NR7$S(O)_2$R7, $(C_1-C_6)$- alkylène-SR7, $(C_1-C_6)$-alkylène-S(O)R7, $(C_1-C_6)$- alkylène-$S(O)_2$R7, $(C_1-C_6)$-alkylène-$S(O)_2$NR7R8, $(C_1- C_6)$-alkylène-COR7, $(C_1-C_6)$-alkylène-COOR7, $(C_1-C_6)$- alkylène-CONR7R8, SR7, S(O)R7, $S(O)_2$R7, S $(O)_2$NR7R8, NR7$S(O)_2$R7, $(C_1-C_6)$-alkylène-$(C_3-C_{10})$-cycloalkyle, $(C_1-C_6)$-alkylène-$(C_6-C_{10})$-aryle, $(C_1-C_6)$-alkylène- hétérocyclyle, $(C_3-C_{10})$-cycloalkyle, $(C_6-C_{10})$-aryle ou hétérocyclyle ;

où les significations pipéridin-4-yle non substitué ou substitué et -CH2-(C6H4)-(C6H5) non substitué ou substitué sont exclues,

R7, R8 signifient, indépendamment l'un de l'autre, H, $(C_1-C_6)$-alkyle, $-CF_3$, $(C_3-C_{10})$-cycloalkyle, $(C_6-C_{10})$- aryle, hétérocyclyle, $(C_1-C_6)$-alkylène-CONR9R10, CONR9R10, $(C_1-C_6)$-alkylène-COOR9, COOR9, COR9, $(C_1-C_6)$-alkylène-COR9 , $(C_1-C_6)$-alkylène-OR9, $(C_1-C_6)$- alkylène-NR9R10, $(C_1-C_6)$-alkylène-SR9, $(C_1-C_6)$- alkylène-S(O)R9, $(C_1-C_6)$-alkylène-$S(O)_2$R9, S(O)R9, $S(O)_2$R9 , $(C_1-C_4)$-alkylène-$(C_6-C_{10})$-aryle ou $(C_1-C_4)$- alkylène-hétérocyclyle ;

R9, R10 signifient indépendamment l'un de l'autre H, $(C_1-C_6)$-alkyle, $(C_1-C_6)$-alkylène- $(C_6-C_{10})$ -aryle, -$(C_6-C_{10})$-aryle, hétérocyclyle ou $(C_1-C_6)$-alkylène- hétérocyclyle ;

R4, R5 signifient, indépendamment l'un de l'autre, H, $(C_1-C_6)$-alkyle ou $(C_3-C_8)$-cycloalkyle, où $(C_1-C_6)$- alkyle ou $(C_3-C_8)$-cycloalkyle peuvent être substitués par F, Cl, Br, I, CN, aryle, hétérocyclyle, $NH_2$, NH$(C_1-C_6)$-alkyle, N($(C_1-C_6)$- alkyle$)_2$, OH, O$(C_1-C_6)$-alkyle, O-aryle, O- hétéroaryle, S$(C_1-C_6)$-alkyle,S(O)$(C_1-C_6)$-alkyle ou $S(O)_2$ $(C_1-C_6)$-alkyle, où tous ces groupes alkyle peuvent à leur tour être substitués par F, Cl, Br ou I ;

R11, R12, R13, R14, R15, R16 signifient, indépendamment l'un de l'autre, H, $(C_1-C_6)$-alkyle, aryle, hétérocyclyle, $(C_3-C_8)$-cycloalkyle, $(C_1-C_4)$- alkylène-O- $(C_1-C_6)$-alkyle, $(C_1-C_4)$-alkylène-S- $(C_1- C_6)$-alkyle, $(C_1-C_4)$-alkylène-NH-$(C_1-C_6)$-alkyle, $(C_1- C_4)$-alkylène-N(alkyle$)_2$, $(C_1-C_4)$-alkylène-$(C_6-C_{10})$- aryle, $(C_1-C_4)$-alkylène-hétérocyclyle, F, Cl, Br, I, CN, COOH, COO$(C_1-C_6)$-alkyle, $CONH_2$, CONH$(C_1-C_6)$- alkyle, CON((C1-C6)-alkyle$)_2$, $CF_3$, ou deux des radicaux R11, R12, R13, R14, R15, R16 forment ensemble un radical $(C_2-C_6)$-alkylène, sur lequel peut être condensé un radical $(C_6-C_{10})$-aryle ou un radical $(C_6-C_{10})$hétérocyclyle et ces radicaux aryle ou hétérocyclyle peuvent être substitués par F, Cl, Br, I, $OCF_3$, $CF_3$, CN, $(C_1-C_6)$ -alkyle, aryle, hétérocyclyle, $(C_3-C_8)$-cycloalkyle, $(C_1-C_4)$- alkylène-O- $(C_1-C_6)$-alkyle, $(C_1-C_4)$-alkylène-S-$(C_1- C_6)$-alkyle, $(C_1-C_4)$-alkylène-NH-$(C_1-C_6)$-alkyle, $(C_1- C_4)$-alkylène-N($(C_1-C_6)$-alkyle$)_2$, $(C_1-C_4)$-alkylène- $(C_6-C_{10})$-aryle, $(C_1-C_4)$-alkylène-hétérocyclyle, COOH, COO$(C_1-C_6)$ -alkyle, $CONH_2$, CONH$(C_1-C_6)$-alkyle, CON($(C_1-C_6)$-alkyle$)_2$, OH, O-$(C_1-C_6)$-alkyle, O-$(C_3- C_6)$-cycloalkyle, S-$(C_1-C_6)$-alkyle, S-$(C_3-C_6)$- cycloalkyle, SO-$(C_1-C_6)$-alkyle, SO-$(C_3-C_6)$- cycloalkyle, $SO_2$-$(C_1-C_6)$-alkyle, $SO_2$-$(C_3-C_6)$- cycloalkyle, $SO_2$-$NH_2$, $SO_2$-NH-$(C_1-C_6)$-alkyle ou $SO_2$- NH-$(C_3-C_7)$-cycloalkyle ;

m vaut 0 ;

n vaut 0 ou 1 ;

ainsi que leurs sels physiologiquement acceptables.

4. Composés de formule I, selon l'une ou plusieurs des revendications 1 à 3, **caractérisés en ce que**

R1 signifie H ;

R2 signifie NR22COR23 ou NR24R25 ;

R22 signifie H ;

R23 signifie $(C_6-C_{10})$-aryle, où le radical aryle peut être monosubstitué ou polysubstitué par F, Cl, Br, I, CN, $NO_2$, SH, OH, $(C_1-C_6)$-alkyle, O-$(C_1-C_6)$- alkyle, S- $(C_1-C_6)$ -alkyle, S(O)-$(C_1-C_6)$-alkyle ou S$(O)_2$-$(C_1-C_6)$-alkyle ;

R24 signifie $(C_1-C_6)$-alkylène-$(C_6-C_{10})$-aryle, où le radical aryle peut être monosubstitué ou polysubstitué par F, Cl, Br, I, CN, $NO_2$, SH, OH, $(C_1-C_6)$ -alkyle, O-$(C_1-C_6)$-alkyle, S-$(C_1-C_6)$-alkyle, S(O)-$(C_1-C_6)$-alkyle ou S$(O)_2$-$(C_1-C_6)$-alkyle ;

R25 signifie H ;

R3 signifie $(C_2-C_{10})$-alcényle

R4, R5 signifient H ;

R11, R12, R13, R14, R15, R16 signifient H ;

n vaut 0 ou 1 ;

ainsi que leurs sels physiologiquement acceptables.

5. Composés selon l'une ou plusieurs des revendications 1 à 4 destinés à une utilisation comme médicament.

6. Médicament contenant un ou plusieurs des composés selon l'une ou plusieurs des revendications 1 à 4.

7. Médicament contenant un ou plusieurs composés selon l'une ou plusieurs des revendications 1 à 4 et au moins une autre substance active.

8. Médicament selon la revendication 7, **caractérisé en ce qu'**il contient comme autre substance active un ou plusieurs agents antidiabétiques, une ou plusieurs substances actives hypoglycémiques, un ou plusieurs inhibiteurs de la HMGCoA-réductase, inhibiteurs de la résorption du cholestérol, agonistes de PPAR gamma, agonistes de PPAR

alpha, agonistes de PPAR alpha/gamma, fibrates, inhibiteurs de MTP, inhibiteurs de la résorption de l'acide gallique, inhibiteurs de CETP, adsorbants polymères de l'acide gallique, inducteurs du récepteur de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), inhibiteurs de lipase, une ou plusieurs insulines, une ou plusieurs sulfonylurées, un ou plusieurs biguanides, méglitinides, une ou plusieurs thiazolidinediones, un ou plusieurs inhibiteurs de l'α-glucosidase, une ou plusieurs substances actives agissant sur le canal potassique des cellules bêta dépendant de l'ATP, un ou plusieurs agonistes de CART, agonistes de NPY, agonistes de MC4, agonistes d'orexine, agonistes de H3, agonistes de TNF, agonistes de CRF, antagonistes de CRF BP, agonistes de l'urocortine, agonistes de β3, antagonistes du récepteur CB1, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, une ou plusieurs hormones de croissance, un ou plusieurs composés libérant une hormone de croissance, agonistes de TRH, modulateurs 2 ou 3 découplant les protéines, agonistes de leptine, agonistes de DA (bromocriptine, doprexine), inhibiteurs de lipase/amylase, modulateurs de PPAR, modulateurs de RXR ou agonistes de TR-β ou une ou plusieurs amphétamines.

**9.** Utilisation des composés selon l'une ou plusieurs des revendications 1 à 4 pour la préparation d'un médicament destiné à abaisser la glycémie.

**10.** Utilisation des composés selon l'une ou plusieurs des revendications 1 à 4 pour la préparation d'un médicament destiné au traitement du diabète de type II.

**11.** Utilisation des composés selon l'une ou plusieurs des revendications 1 à 4 pour la préparation d'un médicament destiné au traitement des troubles du métabolisme lipidique et des hydrates de carbone.

**12.** Utilisation des composés selon l'une ou plusieurs des revendications 1 à 4 pour la préparation d'un médicament destiné au traitement de phénomènes artérioscléreux.

**13.** Utilisation des composés selon l'une ou plusieurs des revendications 1 à 4 pour la préparation d'un médicament destiné au traitement de la résistance à l'insuline.

**14.** Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1122257 A
- EP 0392317 A
- WO 2005021536 A
- US 6221633 B
- WO 9808871 A
- WO 9726265 A
- WO 9903861 A
- EP 200400269 W
- EP 200305815 W
- EP 200305814 W
- EP 200305816 W
- EP 0114531 A
- US 6498156 B
- US 11833 W
- US 11490 W
- DE 10142734
- US 6245744 B
- US 6221897 B
- US 6342512 B
- WO 9741097 A
- EP 0912520 A
- EP 06749 W
- WO 0191752 A
- WO 0063208 A
- WO 0066585 A
- WO 0183451 A
- WO 0228346 A
- WO 9915525 A
- WO 0071549 A
- WO 0109111 A
- WO 0185695 A
- EP 0462884 A
- WO 0040569 A
- WO 0078312 A


### In der Beschreibung aufgeführte Nicht-Patentliteratur


- *Pharmaceutical Research,* 1986, vol. 2 (6), 318
- **Asakawa, A et al.** Cocaine-amphetamine-regulated transcript influences energy metabolism, anxiety and gastric emptying in mice. *M.: Hormone and Metabolic Research,* 2001, vol. 33 (9), 554-558
- **Lee, Daniel W. ; Leinung, Matthew C. ; Rozhavskaya-Arena, Marina ; Grasso, Patricia.** Leptin agonists as a potential approach to the treatment of obesity. *Drugs of the Future,* 2001, vol. 26 (9), 873-881
- **Salvador, Javier ; Gomez-Ambrosi, Javier ; Fruhbeck, Gema.** Perspectives in the therapeutic use of leptin. *Expert Opinion on Pharmacotherapy,* 2001, vol. 2 (10), 1615-1622
- **Zunft H J et al.** Carob pulp preparation for treatment of hypercholesterolemia. *ADVANCES IN THERAPY,* September 2001, vol. 18 (5), 230-6
- **Theodora W. Greene ; Peter G. M. Wuts.** Protective Groups in Organic Synthesis. John Wiley & Sons, Inc, 1999